(19) Europäisches Patentamt
European Patent Office
Office européen des brevets

(11) Numéro de publication: 0 094 305
B1

(12) **FASCICULE DE BREVET EUROPÉEN**

(45) Date de publication du fascicule du brevet: 13.01.88

(51) Int. Cl.⁴: C 07 D 498/06, A 61 K 31/40 // C07D209/90

(21) Numéro de dépôt: 83400908.6

(22) Date de dépôt: 05.05.83

(54) **Nouveaux dérivés de l'acide 9-oxalysergique, leurs sels, procédé et intermédiaires de préparation, application à titre de médicaments et compositions les renfermant.**

(30) Priorité: 12.05.82 FR 8208249

(43) Date de publication de la demande: 16.11.83 Bulletin 83/46

(45) Mention de la délivrance du brevet: 13.01.88 Bulletin 88/2

(84) Etats contractants désignés: AT BE CH DE GB IT LI LU NL SE

(56) Documents cité:
EP-A-0 033 767
EP-A-0 037 784
FR-A-2 273 542

Journal of Medicinal Chemistry 1974, vol. 17, numero 3, p. 312-314
Burger's Medicinal Chemistry 4th Edition, Vol. III, p. 419 (1987)
Fluka Catalogue II, 1978 (English Edition)

(73) Titulaire: ROUSSEL- UCLAF, 35, boulevard des Invalides, F-75007 Paris (FR)

(72) Inventeur: Nedelec, Lucien, 45, boulevard de l'Ouest, F-93340- Le Raincy (FR)
Inventeur: Pierdet, André, 45, avenue François Coppée, F-93250- Villemomble (FR)
Inventeur: Fauveau, Patrick, 40, avenue Camille Desmoulins, F-93190- Livry- Gargan (FR)

(74) Mandataire: Niel, Michel, Département des Brevets ROUSSEL UCLAF B.P. no. 9, F-93230 Romainville (FR)

EP 0 094 305 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne de nouveaux dérivés de l'acide 9-oxalysergique ainsi que leurs sels, le procédé de préparation, l'application à titre de médicaments de ces nouveaux dérivés et les compositions les renfermant, ainsi que des nouveaux intermédiaires.

Les demandes européennes no 37784 et 33767 décrivent des dérivés de l'indolobenzoxazine doués de propriétés antiparkinsonniennes, ant hypertensives et antiprolactines.

L'invention a pour objet de nouveaux dérivés de l'acide 9-oxalysergique, ainsi que leurs sels d'addition avec les acides minéraux ou organiques, caractérisés en ce qu'ils répondent à la formula générale I :

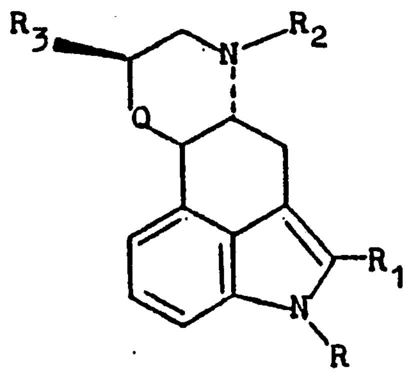

(I)

dans laquelle R représente un atome d'hydrogène ou un radical alcoyle renfermant de 1 à 4 atomes de carbone, R représente un atome d'hydrogène, de chlore ou de brome, $R_2$ représente un atome d'hydrogène, un radical alcoyle renfermant de 1 à 4 atomes de carbone, benzyle ou phénéthyle éventuellement substitués par un ou plusieurs substituants choisis -dans le groupe des atomes d'halogène, des radicaux méthyl, éthyl, méthoxy, hydroxy ou trifluorométhyl ou cycloalcoylalcoyle renfermant de 4 à 7 atomes de carbone et $R_3$ représente un radical alcoyl-thiométhyle, dont l'alcoyle renferme de 1 à 3 atomes de carbone et est linéaire.

Dans la formule générale I et dans ce qui suit, le terme radical alcoyle renfermant de 1 à 4 atomes de carbone désigne de préférence un radical méthyle, éthyle, propyle ou isopropyle; le terme radical cycloalcoylalcoyle désigne, de préférence, un radical cyclopropylméthyl.

Les sels d'addition avec les acides minéraux ou organiques peuvent être par exemple, les sels formé avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfonique, arylsulfoniques, tels que les acides benzène ou paratoluène sulfoniques et arylcarboxyliques.

Parmi les produits objet de l'invention, on peut citer notamment les dérivés répondant à la formule I ci-dessus, ainsi que leurs sels d'addition avec les acides minéraux ou organiques, caractérisés en ce que, dans ladite formule I, R représente un atome d'hydrogène.

Parmi ceux-ci, on peut citer plus particulièrement les dérivés caractérisés en ce que dans ladite formule I, $R_1$ représente un atoms d'hydrogène, de chlore ou de brome, $R_2$ représente un radical alcoyle renfermant de 1 à 4 atomea de carbone, et $R_3$ représente un radical méthylthiométhyle, et tout particulièrement 1a /6a RS (6aα,9β,10aβ/ 4,6,6a,8,9,10a-hexahydro 7-méthyl 9-méthylthiométhyl 7H-indolo (3,4-g,h) 1,4-benzoxazine, ainsi que ses sels d'addition avec les acides minéraux ou organiques.

L'invention a également pour objet un procédé de préparation des dérivés, tels que définis par la formule I ci-deseus, ainsi que de leurs sels, caractérisé en ca que l'on débenzyle le produit de formule II:

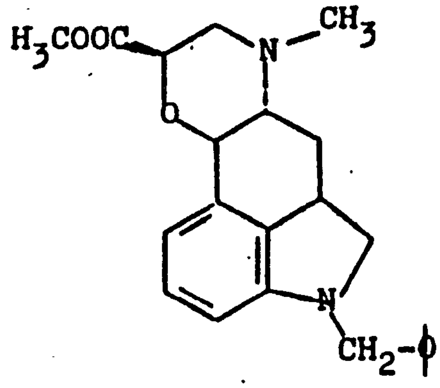

(II)

pour obtenir le produit de formule III

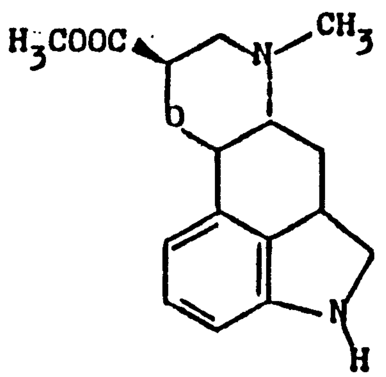

(III)

de formule ($I_A$):

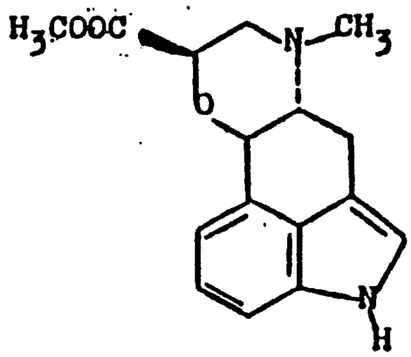

($I_A$)

que l'on réduit pour obtenir un produit de formule ($I_J$):

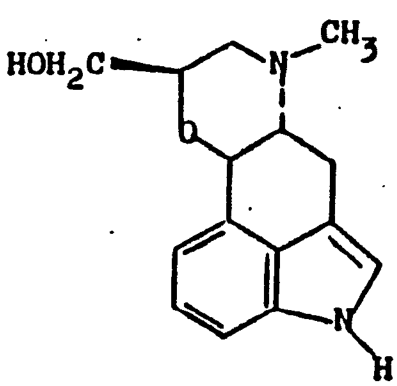

($I_J$)

que l'on fait réagir avec le chlorure de méthane sulfonyle ou le chlorure de paratoluène sulfonyle pour obtenir un produit de formule VI):

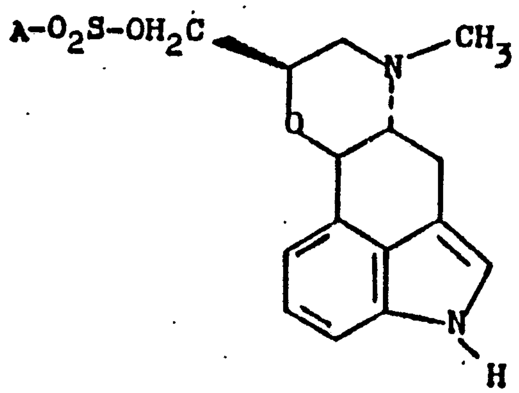

(VI)

dans laquelle A représente un radical méthyle ou para-tolyle, que l'on fait réagir avec un alcoylmercaptan, pour obtenir un produit de formule ($I_K$):

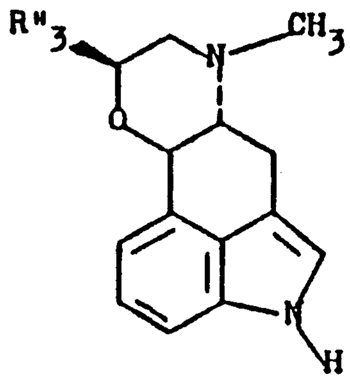

($I_K$)

dans laquelle R"$_3$ représente un radical alcoylthiométhyle que soit l'on isole et si désiré, salifie, soit l'on déméthyle pour obtenir le produit de formule $I_B$

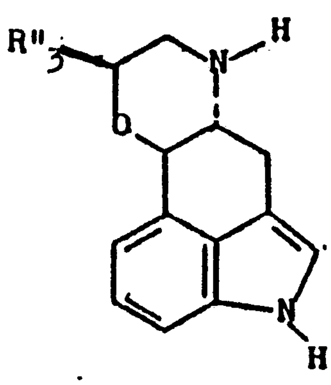

($I_B$)

dans laquelle R"$_3$ a la signification déjà indiquée, que soit l'on isole et si désiré salifie, soit l'on fait réagir avec un agent d'alcoylation pour obtenir un produit de formule $I_C$

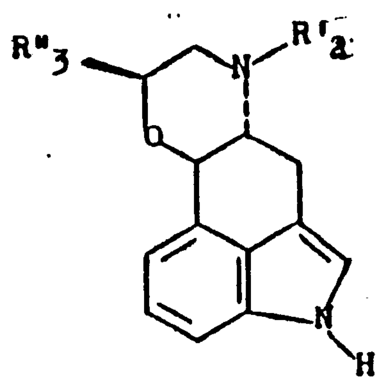

($I_C$)

dans laquelle $R''_3$ a la signification déjà indiquée et $R'_2$ a la signification de $R_2$ déjà indiquée à l'exception de l'hydrogène, que ou bien l'on isole, et, si désiré, salifie, ou blien l'on fait réagir avec un amidure alcalin, puis avec un halogénure d'alcoyle de formula IV

Hal-R' (IV)

dans laquelle Hal représente un atome de chlore, de brome, ou d'iode, et R' a la signification de R déjà indiquée, à l'exception de l'hydrogène, pour obtenir un produit de formule $I_D$

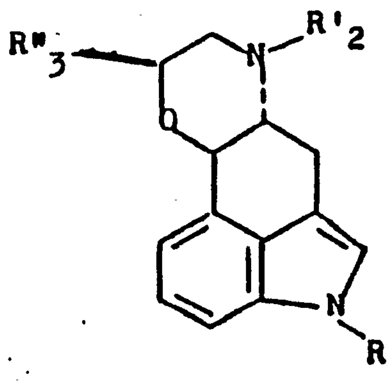

($I_D$)

dans laquelle R', $R''_3$ et $R'_2$ ont la signification déjà indiquée que soit l'on isole et, si désiré, salifie, soit l'on soumet à l'action d'un agent d'halogénation pour obtenir un produit de formule $I_E$

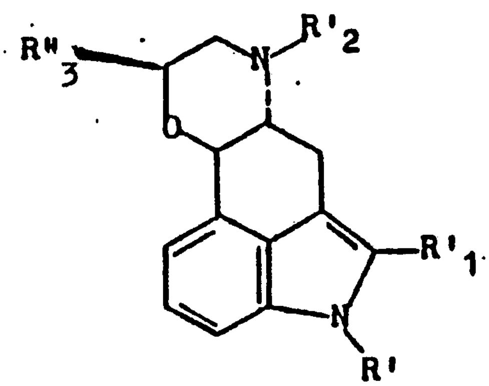

($I_E$)

dans laquelle R', $R''_3$ et $R'_2$ ont le signification déjà indiquée at $R'_1$ représente un atome de chlore ou de brome que l'on isole et salifie si désiré , ou bien l'on soumet ledit produit de formule $I_C$ à l'action d'un agent d'halogénation pour obtenir un produit de formule $I_F$

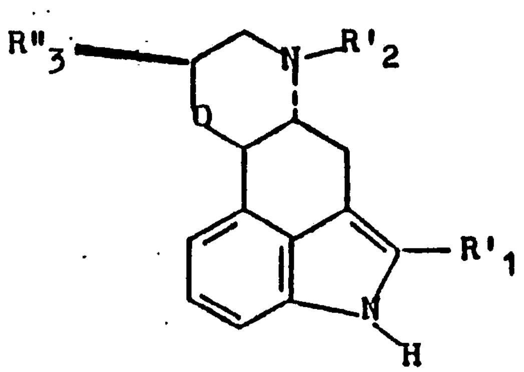

$(I_F)$

dans laquelle $R'_1$, $R''_3$ et $R'_2$ ont la signification déjà indiquée que l'on isole et si désiré, salifie.

La débenzylation du produit de formule II est réalisée de préférence par hydrogénation catalytique. On utilise avantageusement un catalyseur tel que le palladium dans l'acide acétique.

La déshydrogénation du produit de formule III est réalisée de préférence à l'aide d'un oxydant tel que le bioxyde de manganèse ; elle peut également être réalisée par action de palladium, au reflux du xylène.

La déméthylation du produit de formule $I_A$ est réalisée de préférence par action de bromure de cyanogène, suivie d'une réduction telle qu'obtenue par action du zinc dans l'acide acétique.

L'alcoylation du dérivé de formule $I_B$ est réalisée de préférence à l'aide d'un halogénure d'alcoyle, notamment un iodure d'alcoyle, en présence d'un agent de condensation tel qu'un carbonate alcalin. Pour la méthylation, on opère avantageusement par action de formol dans un solvant tel que l'acétonitrile en présence d'un agent réducteur, par exemple le cyanoborohydrure de sodium ou notamment le borohydrure de sodium.

L'halogénure de formule IV peut être un chlorure ou un bromure, mais de préférence un iodure ; on le fait réagir après action, notamment dans l'ammoniaque, d'un amidure alcalin, de préfrénce d'un amidure de sodium, sur le produit de formule $I_C$.

L'halogénation des produits de formule $I_C$ et $I_D$ peut-être réalisée par exemple à l'aide de N-chloro succinimide dans le cas de la chloration ; elle est réalisée à l'aide de N-bromosuccinimide ou, de préférence à l'aide du complexe bromé de la pyrrolidone de formule :

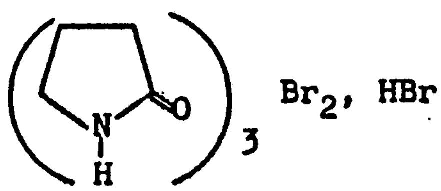

dans le cas de la bromation.

La réduction du produit de formule $I_A$ est réalisée de préférence par action de borohydrure de sodium, au reflux d'un solvant tel que la dioxanne ou le mélange dioxanne-méthanol ; on peut également utiliser d'autres réducteure tels l'hydrure d'aluminium-lithium ou le cyano-borohydrure de sodium.

La réaction du produit de formule $I_J$ avec le chlorure de méthane sulfonyle est réalisée de préférence dans la pyridine à température ambiante.

La réaction du produit de formule VI avec l'alcoyl mercaptan est réalisée de préférence à température ambiante dans un solvant tel que le diméthylacétamide, en présence d'hydrure de sodium.; l'alcoyl mercaptan et de préférence le méthyl mercaptan .

Dans des conditions préférentielles de mise en oeuvre de l'invention, le procédé de préparation ci-dessus décrit est caractérisé en ce que le produit de formule II est préparé par réaction d'un produit de formule VII

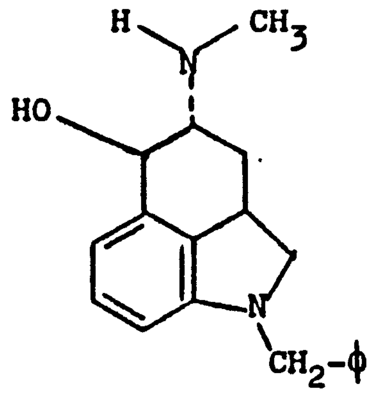

(VII)

avec un glycidate d'alcoyle de formule VIII

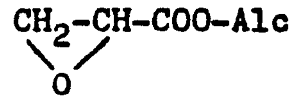

(VIII)

dans laquelle Alc représente un radical alcoyle renfermant de 1 à 4 atomes de carbone, pour obtenir un produit de formule IX

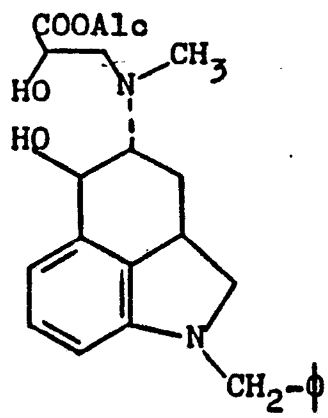

(IX)

dans laquelle Alc a la signification déjà indiquée, que l'on cyclise pour obtenir un produit de formule X

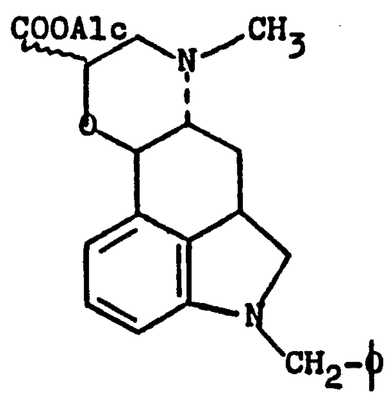

(X)

dans laquelle Alc a la signification déjà indiquée, et le trait ondulé signifie que le substituant est en position 9α ou 9β, dont on épimérise l'isomère 9α, puis estérifie l'acide ainsi obtenu, dans lequel le substituant en 9 est en position 9β pour obtenir ledit produit de formule II.

Le glycidate d'alcoyle de formule VIII est avantageusement le glycidate d'éthyle ; on opère de préférence au reflux d'un alcanol renfermant de 1 à 4 atomes de carbone, de préférence l'alcanol correspondant à l'alcoyle estérifiant l'acide glycidique.

La cyclisation du produit de formule IX est réalisée par action de N-chloro diisopropylamine en présence

d'hexaméthyl phosphoramine ; on peut également opérer par action de tétrachlorure de carbone en présence de triphényl phosphine ou d'hexaméthyl phosphoramine, puis cyclisation du dérivé chloré obtenu par action d'hydrure de sodium dans un solvant tel que le diméthoxy éthane.

L'épimérisation des produits de formule X de structures 9α et 9β est réalisée classiquement, de préférence en milieu basique, par exemple par action d'un alcoolate alcalin, notament l'éthylate de sodium, au reflux pendant 1 à 4 heures;

L'estérification conduisant au produit de formule II est réalisée de préférence par action du diazométhane.

Les dérivés de formule I présentent un caractère basique. On peut avantageugeusement préparer les sels d'addition des dérivés de formule I, en faisant réagir, en proportions sensiblement stoechiométriques, un acide minéral ou organique avec ledit dérivé de formule I. Les sels peuvent être préparés sans isoler les bases correspondantes.

Les dérivés objet de la présente invention, possèdent de très intéressantes propriétés pharmacologiques ; ils sont doués notamment de remarquables propriétés agonistes dopaminergiques, inhibitrices de la sécrétion de prolactine, sérotoninergiques et antihypertensives. Certains dérivés sont par ailleurs doués de propriétés vasodilatatrices et de propriétés anti-anoxiques.

Ces propriétés sont illustrées plus loin dans la partie expérimentale.

Ces propriétés justifient l'utilisation des dérivés de l'acide 9-oxalysergique, ainsi que de leurs sels pharmaceutiquement acceptables à titre de médicaments.

La présente demande a ainsi également pour objet l'application à titre de médicaments, des dérivés de l'acide 9-oxalysergique, tels que définis par la formule I, ainsi que de leurs sels d'addition avec les acides pharmaceutiquement acceptables.

Parmi les médicaments, objet de l'invention, on retient de préférencs les médicaments caractérisés en ce qu'ils sont constitués par les nouveaux dérivés de l'acide 9-oxalysergique répondant à la formule I, dans laquelle R représente un atome d'hydrogène, ainsi que par leurs sels d'addition avec les acides pharmaceutiquement acceptables.

Parmi ceux-ci, on retient notamment ceux répondant à la formule I, dans laquelle $R_1$ représente un atome d'hydrogène, de chlore ou de brome, $R_2$ représente un radical alcoyle renfermant de 1 à 4 atomes de carbone, et $R_3$ représente un radical méthylthiométhyle, ainsi que leur sels d'addition avec les acides pharmaceutiquement acceptables.

Parmi ces derniers, on retient tout particulièrement

1a /6a RS (6aα, 9β, 10aβ)/ 4,6,6a,8,9,10a-hexahydro 7-méthyl 9-méthylthiométhyle 7H-indolo (3,4-g,h) 1,4-benzoxazine, ainsi que ses sels d'addition avec les acides pharmaceutiquement acceptables.

Les médicaments selon l'invention trouvent leur emploi, par exemple dans le traitement des syndromes neurologiques d'origine extrapyramidale, par exemple dans le traitement de la maladie de Parkinson et dans le traitement des syndromes parkinsonniens post-encéphalitiques ; ils peuvent également être utilisés dans le traitement de l'hypersécrétion de prolactine par l'antéhypophyse, par exemple dans le traitement de l'hypogonadisme de la femme ou de l'homme. Ils peuvent aussi être utilisés dans le traitement de l'hypertension artérielle essentielle, de l'hypertension de la cinquantaine, de la ménopause, du diabétique, de l'obèse et du pléthorique, ainsi que dans le traitement de l'hypertension artérielle du sujet agé, ou atteint d'artériosclérose, et dans le traitement de l'hypertension d'origine rénale. Ils peuvent aussi être utilisés dans le traitement de le sénesence cérébrale ou des manifestatione liées à une hypoxie cérébrale.

La dose usuelle, variable selon le produit utilisé, le sujet traité et l'affection en cause, peut être par exemple, de 5 mg à 200 mg par jour, par vole orale, chez l'homme, du produit de l'exemple 2.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule I et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

**Prèparation 1** :6a RS (6aα, 9β, 10aβ)/ 4,6,6a,8,9,10a-héxahydro 7-méthyl-7H-indolo / 3,4-g,h/ (1,4)-benzoaxzine 9-carboxylate de méthyle et son chlorhydrate.

*Stade A: /6a RS (6aα*,9β,10aβ)/7-méthyl 4,5,5a,6,6a,8,9, 10a-octahydro -7H-indolo / 3,4-g,h/ (1,4)-benzoxazine-9-carboxylate de méthyle.

On hydrogène pendant 4 heures 12,75 g de /(6a RS (6aα,9β, 10aβ)/ 4,5,5a,6,6a,8,9,10a-octahydro 7-méthyl-4-phényl-méthyl 7H-indolo /3,4-g,h/ (1,4)-benzoxazine 9β-carboxylate de méthyle dans 600 cm$^3$ d'acide acétique en présence de 3,2 g de palladium à 10 % sur charbon actif. On filtre, lave à l'acide acétique, amène à sec à 30-35°C sous pression réduite, ajoute 200 cm$^3$ d'eau, alcalinise à l'ammoniaque jusqu'à pH ≥ 9, extrait au chlorure de méthylène, lave à l'eau, sèche, amène à sec sous pression réduite, ajoute quelques cm$^3$ d'éther, essore,

sèche à 50° C sous pression réduite et obtient 8,28 g du produit attendu. F ≃ 178° C.

Par chromatographie sur silice des liqueurs mères (éluant chloroforme-méthanol 95- 5) on récupère encore 0,475 g de produit. F ≃ 178° C.

*Stade B : /6a RS (6aα,9β,10aβ)/* 4,6,6a,8,9,10a-hexahydro 7-méthyl 7H-indolo /3,4-g,h/ (1,4)-benzoxazine 9-carboxylate de méthyle.

On ajoute 33,5 g de bioxyde de manganèse, à une solution de 8,4 g du produit obtenu au stade A dans 840 $cm^3$ de chlorure de méthylène, agite sous atmosphère inerte pendant 16 heures, filtre, lave, amène à sec sous pression réduite, ajoute quelques $cm^3$ d'éther, essore, sèche, et obtient en 2 jets 3,9 g puis 0,91 g de la base attendue (F ≃ 174°C) ainsi que 1,1 g supplémentaire par chromatographie sur silice des liqueurs mères (F ≃ 174°C).

Formation du chlorhydrate :

On dissout à chaud 2 g de la base obtenue ci-dessus dans 50 $cm^3$ de méthanol, ajoute 5 $cm^3$ de méthanol chlorhydrique 2N, essore, lave avec du méthanol, sèche à 50° C sous pression réduite, recristallise dans le méthanol et obtient 1,78 g du produit attendu. F ≃ 248° C (avec décomposition).

Analyse : $C_{16}H_{18}N_2O_3$, HCl = 322,78

| | | | | | | | | |
|---|---|---|---|---|---|---|---|---|
| Calculé : | C % | 59,53 | H % | 5,93 | N % | 8,68 | Cl % | 10,98 |
| Trouvé : | | 59,3 | | 6,1 | | 8,5 | | 10,8 |

**Préparation 2** : /6a RS (6aα,9β,10aβ)/4,6,6a,8,9,10a-hexahydro 7-méthyl 7H-indolo /3,4-g,h/ (1,4)-benzoxazine 9-méthanol et son chlorhydrate.

On porte au reflux pendant 1 heure sous agitation et atmosphère inerte, 2,2 g de la base obtenue à la préparation 1 30 $cm^3$ de dioxanne et 15 $cm^3$ de méthanol, avec 2,2 g de borohydrure de sodium à 95 %, refroidit, ajoute 150 $cm^3$ d'eau, extrait au chlorure de méthylène, lave à l'eau, sèche, filtre, amène à sec sous pression réduite, ajoute quelques $cm^3$ d'éther, essore, sèche à 50°C sous pression réduite, et obtient en 2 jets 1,82 g puis 0,08 g de la base attendue (F ≃ 208° C).

Formation du chlorhydrate :

On dissout à chaud 2 g de la base obtenue ci-dessus dans 80 $cm^3$ de méthanol, ajoute 5 $cm^3$ de méthanol chlorhydrique 2N, laisse cristalliser pendant 16 heures, essore, lave à l'éther, sèche à 50°C sous pression réduite et obtient 1,97 g du chlorhydrate attendu F = 320° C.

Analyse : $C_{15}H_{18}N_2O_2$, HCl = 294,77

| | | | | | | | | |
|---|---|---|---|---|---|---|---|---|
| Calculé : | C % | 61,11 | H % | 6,50 | N % | 9,50 | Cl % | 12,03 |
| Trouvé: | | 61,0 | | 6,5 | | 9,3 | | 12,2 |

**Préparation 3** : Acide /6a RS (6aα,9β,10aβ)/ 4,6,6a,8,9,10a-hexahydro 7-méthyl 7H-indolo /3,4-g,h/ (1,4)-benzoxazine-9-carboxylique.

On porte au reflux pendant 3 heures, sous atmosphère inerte 1,61 g de la base obtenue à la préparation 1, dans 150 $cm^3$ de méthanol, avec une solution de 520 mg de sodium dans 20 $cm^3$ de méthanol, amène à sec sous pression réduite, ajoute quelques $cm^3$ d'eau, ajoute de l'acide acétique jusqu'à pH ≃ 6,4, essore, lave à l'eau puis à l'acétone, sèche, et obtient 1,5 g du produit attendu.

**Préparation 4** : /6a RS (6aα,9β,10aβ)/ N,N-diéthyl 4,6,6a,8,9,10-hexahydro 7-méthyl 7H-indolo /3,4-g,h/ (1,4) benzoxazine-9-carboxamide et son chlorhydrate.

On ajoute 1,3 $cm^3$ de tributylamine et 0,63 $cm^3$ de chloroformiate d'isobutyle à une solution de 1,22 g de l'acide obtenu à la préparation 3, dissous dans 30 $cm^3$ de dioxanne et 30 $cm^3$ de diméthylformamide. On agite pendant 1 heure à 20°C sous atmosphère inerte et verse cette solution dans 30 $cm^3$ de dioxanne contenant 2,7 $cm^3$ de diéthylamine, et agite pendant 1 heure sous atmosphère inerte. On ajoute 100 $cm^3$ d'eau, extrait au chlorure de méthylène, lave à l'eau, sèche, amène à sec sous pression réduite, et recueille 1,03 g de la base attendue F ≃ 210°C, puis 150 mg (F ≃ 210°C) après chromatographie sur silice des liqueurs mères (éluant : chloroforme-méthanol 95/5)

Formation du chlorhydrate :

On dissout à chaud 1,18 g de la base ci-dessus dans 50 $cm^3$ de méthanol, refroidit, ajoute à +10° C, +15° C, 5 $cm^3$ de méthanol chlorhydrique ≃ 2N, laisse cristalliser pendant 16 heures, essore, lave, sèche sous pression réduite et obtient 1,07 g du produit attendu, F > 250° C.

Analyse : $C_{19}H_{25}N_3O_2$, HCl = 363,87

| | | | | | | | | |
|---|---|---|---|---|---|---|---|---|
| Calculé : | C % | 62,70 | H % | 7,20 | N % | 11,55 | Cl % | 9,74 |
| Trouvé : | | 62,4 | | 7,2 | | 11,5 | | 10,0 |

Le /6a RS (6aα,9β,10aβ)/ 7-méthyl 4,5,5a,6,6a,8,9,10a-octahydro-4-phénylméthyl 7H-indolo /3,4-g,h/ (1,4)-benzoxazine-9-carboxylate de méthyle utilisé au départ de la préparation 1 peut être préparé comme suit :

*Stade 1 : (4 RS trans) 2-hydroxy 3/(5-hydroxy-1-phénylméthyl 1,2,2a,3,4,5-hexahydrobenz /c,d/ indol-4-yl) méthylamino/ propanoate d'éthyle.*

On porte pendant 4 heures au reflux sous atmosphère inerte 24,2 g de (4 RS trans) 4-méthylamino 1,2,2a,3,4,5-hexahydro 1-(phénylméthyl) benz (c,d) indol-5-ol dans 1,2 l d'éthanol avec 19,2 g de glycidate

d'éthyle et amène à sec sous pression réduite. On purifie l'huile obtenue par chromatographie sur silice (éluant: benzène-acétate d'éthyle 7/3) et obtient 28,2 g du produit attendu.

*Stade 2 : /6a RS (6aα,9β,10aβ)/* et /6a RS (6aα,9β,10aβ)/ 7-méthyl 4,5,5a,6,6a,8,9,10a-octahydro 4-(phényl méthyl) 7H-indolo /3,4-g,h)(1,4)benzoxazine-9 -carboxylate d'éthyle/

On refroidit à -40/-45°C une solution de 22,6 g du produit obtenu au stade précédent et de 17,25 g de N-chloro diisopropylamine dans 200 $cm^3$ de chlorure de méthylène. On ajoute goutte à goutte en 15 mn, 31,8 $cm^3$ d'héxaméthyl phosphoramine sous atmosphère inerte, agite pendant 15 mn, laisse pendant 1 heure revenir à température ambiante et verse dans 250 $cm^3$ d'eau. On décante, extrait avec une solution aqueuse d'acide chlorhydrique 2N, alcalinise par de la lessive de soude, extrait au chlorure de méthylène, lave à l'eau, sèche, amène à sec sous pression réduite. On ajoute quelques $cm^3$ d'éther isopropylique, essore, sèche et obtient 9,6 g d'isomère 9α du produit cherché, F ≃ 156°C.

On purifie les liqueurs mères par chromatographie sur silice (éluant : benzène-acétate d'éthyle 7/3), et obtient à nouveau 2,2 g d'isomère 9α, F ≃ 157°C et 3,4 g d'isomère 9β (F ≃ 110° C).

*Stade 3 : /6a RS (6aα,9β,10aβ)/* 7-méthyl 4,5,5a,6,6a,8,9,10a-octahydro 4-(phénylméthyl) 7H-indolo /3,4-g,h/ (1,4) benzoxazine 9β-carboxylate de méthyle.

a) Epimérisation :

On porte au reflux pendant 1 heure sous atmosphère inerte 14,5 g du mélange d'isomères 9α et 9β obtenu ci-dessus dans 1 l d'éthanol, avec une solution de 3,6 g de sodium dans 200 $cm^3$ d'éthanol. On amène à sec sous pression réduite, ajoute 50 $cm^3$ d'eau, ajoute de l'acide chlorhydrique concentré jusqu'à pH=6,5 et distille à sec sous pression réduite à ~50°C.

b) Estérification :

On reprend le résidu obtenu par 1,2 l de chlorure de méthylène et 50 $cm^3$ de méthanol, refroidit à +5, +10°C, ajoute goutte à goutte 300 $cm^3$ d'une solution de diazométhane dans le chlorure de méthylène à 12 g/l, laisse pendant 16 heures à 4°C, détruit l'excès de diazométhane par quelques gouttes d'acide acétique, lave à la soude 2N, à l'eau, sèche, amène à sec sous pression réduite, ajoute quelques $cm^3$ d'éther, essore, sèche et obtient 11,8 g du produit attendu, F ≃ 127°C (130°C après recristallisation dans l'éther isopropylique).

Analyse : $C_{24}H_{28}N_2O_3$ = 378,46

| | | | | | | |
|---|---|---|---|---|---|---|
| Calculé : | C % | 72,99 | H % | 6,92 | N % | 7,40 |
| Trouvé : | | 72,7 | | 7,0 | | 7,3 |

**Préparation du 4RS trans 1,2,2a,3,4,5-hexahydro 4-méthylamino 1-(phénylméthyl) benz (c,d) indol-5-ol de départ du stade 1 :**

1. On ajoute lentement sous atmosphère inerte et agitation 26 g de 4RS trans 4-amino 1-benzoyl 1,2,2a,3,4,5-hexahydro benz (c,d) indol-5-ol, à 26 g d'hydrure d'aluminium lithium et 13 g de chlorure d'aluminium dans 800 $cm^3$ de dioxanne, porte 2 heures au reflux, refroidit au bain de glace, ajoute goutte à goutte 300 $cm^3$ de tétrahydrofurane hydraté à 20 % et 300 $cm^3$ de soude 2N, essore le précipité formé, lave au chlorure de méthylène, reprend le filtrat par 1,5 l de chlorure de méthylène, lave à l'eau, sèche, évapore à sec et récupère 20 g de 4RS trans 4-amino 1,2,2a,3,4,5-hexahydro 1-(phénylméthyl) benz (c,d) indol-5-ol fondant à 166°C après recristallisation dans le chlorure de méthylène.

2. On dissout 10 g du produit obtenu ci-dessus dans 100 $cm^3$ de chloroforme et 10 $cm^3$ de lessive de soude, refroidit à 0°,+ 5°C, ajoute 25 $cm^3$ d'eau, puis goutte à goutte 4 $cm^3$ de chloroformiate de méthyle, on agite pendant 30 minutes à température ambiante, extrait au chlorure de méthylène à 10 % de méthanol, lave à l'eau saturée en chlorure de sodium, sèche, évapore à sec, et obtient 11,8 g d'un produit utilisé tel quel pour le stade suivant : F=200°C.

3. On met en suspension 11,7 g d'hydrure d'aluminium-lithium dans 250 $cm^3$ de tétrahydrofurane, refroidit à 0° - 5°C, ajoute en 10 minutes 11,7 g du produit obtenu ci-dessus en solution dans 250 $cm^3$ de tétrahydrofurane, après 2 heures de reflux, ajoute 250 $cm^3$ de tétrahydrofurane à 20 % d'eau, extrait au chlorure de méthylène, lave, sèche, évapore à sec et obtient 10 g du produit brut.

On dissout le produit brut ci-dessus dans 100 $cm^3$ de chlorure de méthylène, agite pendant 20 minutes avec du charbon actif, filtre, rince au chlorure de méthylène, ramène le volume à environ 100 $cm^3$ , ajoute 100 $cm^3$ d'éther isopropylique, laisse cristalliser 30 minutes à température ambiante, 30 minutes au bain de glace et obtient 5,5 g du produit attendu, F=148°C.

**Prèparation 5** : /6a RS (aα, 9β, 10aβ)/ N,N-diéthyl 4,6,6a,8,9,10a-hexahydro 5-bromo 7-méthyl 7H-indolo (3,4-g,h) 1,4-benzoaxazine 9-carboxamide et son chlorhydrate.

On dissout 6,43 g de produit obtenu à la préparation 4 dans 1,6 litre de dioxanne et ajoute en 20 minutes, à 25°C, 14,6 g d'hydrotribromure de pyrrolidone en solution dans 1,8 litre de dioxanne. On maintient sous agitation pendant 30 minutes, évapore le solvant à 35°C maximum et dissout le résidu dans du chlorure de méthylène. On lave la solution avec une solution aqueuse saturée de bicarbonate de sodium, puis à l'eau, sèche et concentre à sec. On chromatographie le résidu sur silice en éluant au mélange cyclohexane-chloroforme-triéthylamine (4,4,2), le cristallise dans l'éther et obtient 5,7 g de produit attendu. F > 250°C.

**Formation du chlorhydrate.**

On met le produit obtenu ci-dessus en suspension dans 100 $cm^3$ de méthanol, ajoute sous agitation et en refroidissant, 20 $cm^3$ d'une solution méthanolique d'acide chlorhydrique 1,4M, maintient sous agitation pendant 2 heures à 20°C, essore les cristaux, les lave au méthanol et à l'éther et les sèche. On obtient 4,38 g de

produit attendu. F > 250°C.

Analyse : $C_{19}H_{24}Br\ N_3\ O_2$ HCl

| | | | | | | | | | | |
|---|---|---|---|---|---|---|---|---|---|---|
| Calculé : | C % | 51,54 | H % | 5,69 | N % | 9,49 | Cl % | 8,01 | Br % | 18,05 |
| Trouvé : | | 51,8 | | 5,7 | | 9,5 | | 8,5 | | 17,7 |

**Exemple 1** : /6a RS (6aα,9β,10aβ)/ 4,6,6a,8,9,10a-hexahydro 7-méthyl 9-méthylthiométhyl 7H-indolo (3,4-g,h) 1,4-benzoxazine et son chlorhydrate.

*Stade A : /6a RS (6aα,9β,10aβ)/* 4,6,6a,8,9,10a-hexahydro 7-méthyl 7H-indolo (3,4-g,h) 1,4-benzoxazine 9-paratoluène sulfonate méthanol.

On mélange 6,6 g de produit obtenu à la préparation de pyridine puis ajoute 9,5 g de chlorure de tosyle dans 125 cm$^3$ de pyridine. On maintient sous agitation à 20°C pandant 17 heures, ajoute du chlorure de méthylène, lave avec une solution aqueuse saturée de bicarbonate de sodium, sèche et évapore à sec. On chromatographie le résidu sur silice en éluant au mélange chlorure de méthyle-méthanol (95-5) et obtient 7,7 g de produit attendu que l'on recristallise dans le mélange éther isopropylique-chlorure de méthylène. F = 180°C.

*Stade B : /6a RS (6aα,9β, 10aβ)/* 4,6,6a,8,9,10a-hexahydro 7-méthyl 9-méthylthiométhyl 7H-indolo (3,4-g,h) 1,4-benzoxazine et son chlorhydrate.

On condense 50 cm$^3$ de métylmercaptan et ajoute 150 cm$^3$ de diméthylacétamide à 0°C puis 12,6 g d'hydrure de sodium à 50% dans l'huile minérale. On ajoute à 20°C en 15 minutes 6,6 g de produit obtenu au stade A dans 40 cm$^3$ de diméthylacétamide, laisse 2 heures sous agitation, verse dans de l'eau glacée, extrait au chlorure de méthylène, lave la phase organique à l'eau, sèche et évapore le solvant. On chromatographie le résidu sur silice en éluant au mélange chlorure de méthylène-méthanol (95-5) et obtient 4,2 g de produit attendu que l'on recristallise dans le chlorure de méthylène. F = 150°C.

Formation du chlorhydrate.

On dissout 3,96 g de produit obtenu ci-dessus dans 150 cm$^3$ de chlorure de méthylène, ajoute 7 cm$^3$ d'une solution d'acide chlorhydrique dans l'éther éthylique, laisse cristalliser à 20°C, essore, lave les cristaux au chlorure de méthylène et sèche. On obtient 4,1 g de produit attendu que l'on recristallise dans le méthanol. F = 250°C. (Décomposition).

Analyse : $C_{16}\ H_{10}\ N_2$ OS, HCl

| | | | | | | | | | | |
|---|---|---|---|---|---|---|---|---|---|---|
| Calculé : | C % | 59,15 | H % | 6,52 | N % | 8,62 | S % | 9,87 | Cl % | 10,91 |
| Trouvé : | | 58,7 | | 6,6 | | 8,7 | | 9,8 | | 10,9 |

**Exemple 2**:

On a préparé des comprimés répondant à la formule:

- Chlorhydrate de /6a RS (6aα,9β,10a)/ 4,6,6a,8,9,10a-hexahydro 7-méthyl 9-méthylthiométhyl 7H-indolo (3,4-g,h) 1,4,benzoxazine...5 mg
- Excipient q.s. pour un comprimé terminé à ...100 mg.

(détail de l'excipient : lactose, amidon, talc, stéarate de magnésium).

**Etude pharmacologique** :

**1. Comportement de rotation après lésion unilatérale du faisceau nigrostriatal par la 6-hydroxydopamine.**

*Technique*

La lésion est effectuée chez des rats mâles de 220 g environ par injection unilatérale dans le faisceau dopaminergique nigrostrié, de 8µg de 6-hydroxydopamine en solution à 2 µg/µl (U.Ungerstedt, Acta Physiol. Scand. 1971, 82, suppl. 367, 69-93).

Chez de tels animaux, les agonistes dopaminergiques directs, tels que l'apomorphine, administrée par voie générale entraînent un comportement de rotation dans la direction contralatérale au côté lésé.

Le composé étudié est administré au moins 5 semaines après la lésion. Les animaux sont placés dans un rotomètre automatisé qui permet de compter le nombre de rotations effectuées par chaque animal dans les deux sens.

Les produits de l'exemple 1 administré par voie intrapéritonéale provoquent des rotations contralatérales dès la dose de 0,5 mg/kg.

**2. Détermination de l'activité hypotensive.**

L'activité hypotensive a été étudiée sur des rats mâles de souche WISTAR pesent 300 g environ et anesthésiés au nembutal (50 mg/kg par voie intraveineuse).

Le produit testé a été administré par voie intraveineuse dans la veine jugulaire.

La pression artérielle carotidienne a été mesurée avant et après administration du produit testé.

Le tableau ci-après indique les variations exprimées en pourcentage de la pression artérielle après administration du produit testé par rapport à la pression artérielle témoin initiale.

| Produits de l'exemple | Dose mg/kg | Variation % de la pression artérielle 1 mn après administration | 5 mn | 10 mn | 30 mn |
|---|---|---|---|---|---|
| 1 | 1 | - 26 | - 35 | - 39 | - 40 |
| | 0,1 | - 10 | - 11 | - 12 | - 16 |

3. **Etude de l'activité des produits sur le débit sanguin cérébral chez le rat éveillé.**

**PRINCIPE**

Le débit sanguin cérébral a été mesuré par la technique de la clairance de l'hydrogène, selon la méthode polarographique d'Auckland (Circulation Research (1964) 14 p. 164-187).

Les électrodes utilisées pour la détermination de la concentration tissulaire d'hydrogène, sont obtenues par l'introduction d'un fil de platina de 125 µm de diamètre dans un tube de verre éffilé, suivie d'une platinisation de la pointe par électrolyse d'une solution aqueuse à 5 % d'acide chloroplatinique.

Les électrodes sont portées à un potentiel de +0,2 V par rapport à une électrode de référence en argent. L'intensité du courant engendre par l'oxydation de l'hydrogène est mesurée au moyen d'un nanoampèremètre et enregistrée sur un inscripteur potentiometrique.

**PROTOCOLE EXPERIMENTAL**

Les essais ont été réalisés sur des rats mâles Wistar d'un poids moyen compris entre 300 et 430 g, en situation libre dans une boîte de dimensions 20 x 20 cm, h = 24 cm. L'électrode à hydrogène est implantée dans le cortex frontal droit, à une profondeur de 0,5 mm, avec une antériorité de 2,0 mm, et une latéralité de 2,0 mm par rapport au bregma. L'implantation est effectuée sous anesthésie générale (pentobarbital sodique 50 mg/kg i.p.), quelques jours avant le début de l'expérimentation. L'électrode de référence est introduite sur la dure mère dans la région symétrique du cortex contralatéral.

Avant administration du produit par voie intrapéritonéale, le débit sanguin est mesuré à quatre reprises toutes les quinze minutes. L'analyse de variance des valeurs avant produit permet de s'assurer que celles-ci sont homogènes. La moyenne de ces 4 valeurs est alors prise comme valeur de débit avant produit.

Le débit sanguin est également mesuré 5 mn, 15 mn, 30 mn, 45 mn et 60 mn après administration du produit par voie intrapéritonéale. Les valeurs sont exprimées en pourcentage d'augmentation du débit sanguin cérébral.

| Produit de l'exemple | Dose mg/kg | Variation du débit sanguin en % aprés administration du produit 5 mn | 15 mn | 30 mn | 45 mn | 60 mn |
|---|---|---|---|---|---|---|
| 1 | 10 | + 53 | + 70 | + 75 | + 22 | + 35 |

4. **Etude de la toxicité aiguë.**

On a évalué les doses létales $DL_0$ des différents composés testés après administration par voie intrapéritonéale chez la souris.

On appelle $DL_0$ la dose maximale ne provoquant aucune mortalité en 8 jours.

Les résultats obtenus sont les suivants :

| Produits de l'exemple | DL en mg/kg |
|---|---|
| 1 | ⩾ 400 |

**Revendications** pour les Etats contractants BE CH DE GB IT LI LU NL SE

1. Nouveaux dérivés de l'acide 9-oxalysergique, ainsi que leurs sels d'addition avec les acides minéraux ou organiques caractérisés en ce qu'ils répondent à la formule générale I

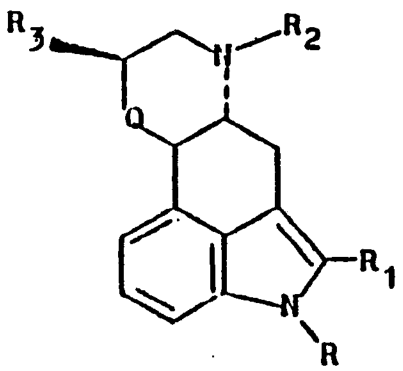

(I)

dans lauelle R représente un atome d'hydrogène ou un radical alcoyle renfermant de 1 à 4 atomes de carbone, $R_1$ représente un atome d'hydrogène, de chlore ou de brome, $R_2$ représente un atome d'hydrogène, un radical alcoyle renfermant de 1 à 4 atomes de carbone, benzyle ou phénéthyle éventuellement substitués par un ou plusieurs substituants choisis dans le groupe des atomes d 'halogène, des radicaux méthyl, éthyl, hydroxy ou trifluorométhyl, ou cycloalcoylalcoyle renfermant de 4 à 7 atomes de carbone et $R_3$ représente un radical alcoyl-thiométhyle, dont l'acoyle renferme de 1 à 3 atomes de carbone et est linéaire.

2. Nouveaux dérivés de l'acide 9-oxalysergique, ainsi que leurs sels d'addition avec les acides minéraux ou organiques, caractérisés en ce qu'ils répondent à la formule I de la revendication 1, dans laquelle R représente un atome d'hydrogène.

3. Nouveaux dérivés de l'acide 9-oxalysergique selon la revendication 2, ainsi que leurs sels d'addition avec les acides minéraux ou organiques, caractérisés en ce que $R_1$ représente un atome d'hydrogène, de chlore ou de brome, $R_2$ représente un radical alcoyle renfermant de 1 à 4 atomes de carbone et $R_3$ représente un radical méthylthiométhyle.

4. Le produit de formule I selon la revendication 1 dont le nom suit :

- 1a /6a RS (6aα,9β,10aβ)/4,6,6a,8,9,10a-hexahydro 7-méthyl 9-méthylthiométhyl 7H-indolo (3,4-g,h) 1,4-benzoxazine, ainsi que ses sels d'addition avec les acides minéraux ou organiques.

5.) Procédé de préparation des dérivés de l'acide 9-oxalysergique tels que définis par la formule I de la revendication 1, ainsi que de leurs sels, caractérisé en ce que l'on débenzyle produit de formule II

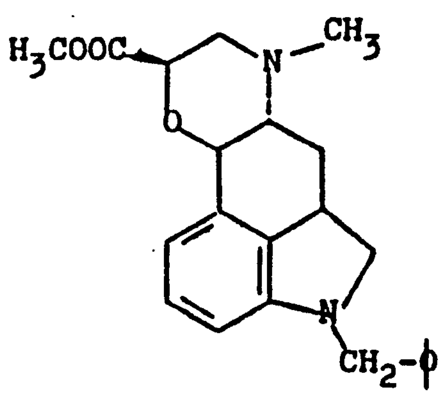

(II)

pour obtenir le produit de formule III

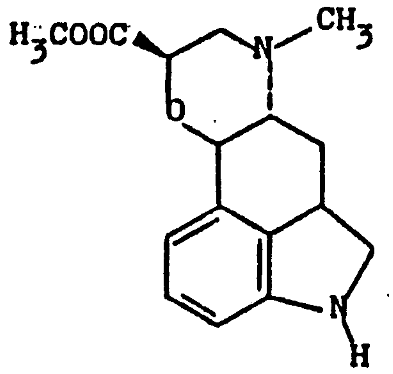

(III)

que l'on soumet à une déshydrogénation pour obtenir le produit de formule ($I_A$) :

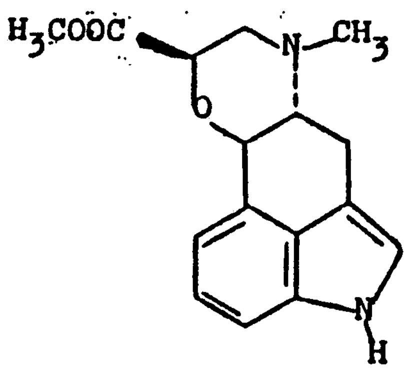

($I_A$)

que l'on réduit pour obtenir un produit de formule ($I_J$) :

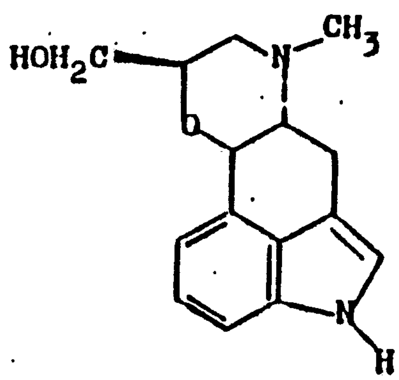

($I_J$)

que l'on fait réagir avec le chlorure de méthane sulfonyle ou le chlorure de paratoluène sulfonyle pour obtenir un produit de formule (VI)

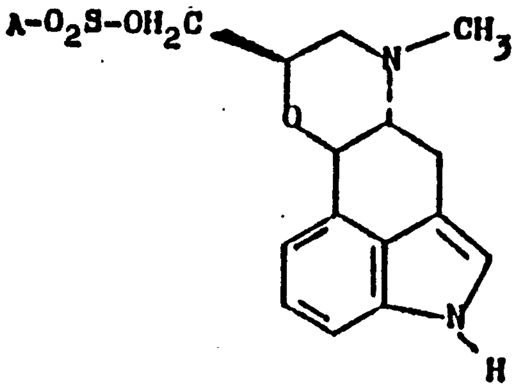

(VI)

dans laquelle A représente un radical méthyle ou para-tolyle, que l'on fait réagir avec un alcoylmercaptan, pour obtenir un produit de formule ($I_K$):

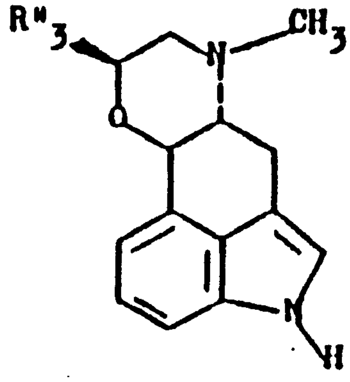

($I_K$)

dans laquelle R"$_3$ représente un radical alcoylthiométhyle que soit l'on isole et si désiré, salifie, soit l'on déméthyle pour obtenir le produit de formule $I_B$

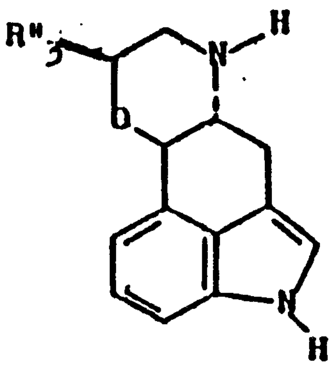

($I_B$)

dans laquelle R"$_3$ a la signification déjà indiquée, que soit l'on isole et si désiré salifie, soit l'on fait réagir avec un agent d'alcoylation pour obtenir un produit de formule $I_C$

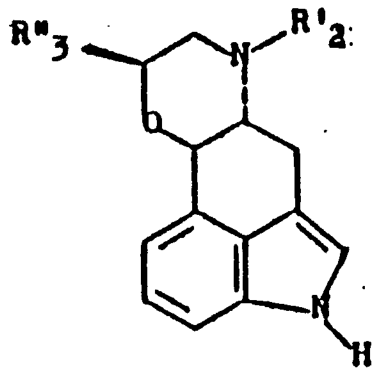

$(I_C)$

dans laquelle $R''_3$ a la signification déjà indiquée et

R'2 a la signification de $R_2$ déjà indiquée à l'exception de l'hydrogène, que ou bien l'on isole, et, si déairé, salifie, ou bien l'on fait réagir avec un amidure alcalin, puis avec un halogénure d'alcoyle de formule IV

Hal-R' (IV)

dans laquelle Hal représente un atome de chlore, de brome, ou d'iode, et R' a la signification de R déjà indiquée, à l'exception de l'hydrogène, pour obtenir un produit de formule $I_D$

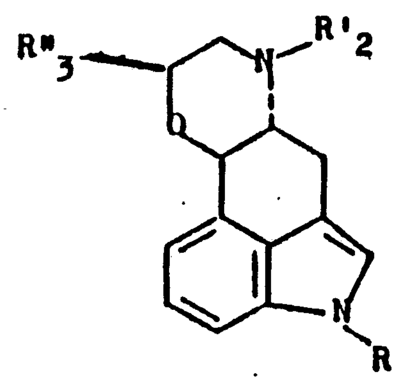

$(I_D)$

dans laquelle R', $R''_3$ et $R'_2$ ont la signification déjà indiquée que soit l'on isole et, si désiré, salifie, soit l'on soumet à l'action d'un agent d'halogénation pour obtenir un produit de formule $I_E$

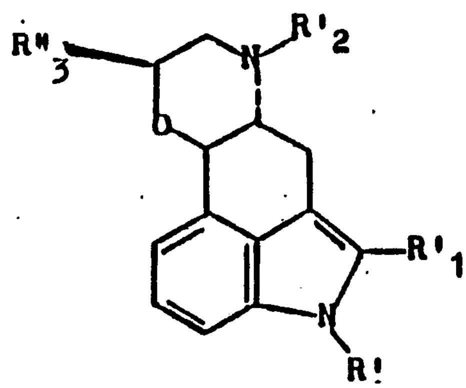

$(I_E)$

dans laquelle R' , R'' et $R'_2$ ont la signification déjà indiquée et $R'_1$ représente un atome de chlore ou de brome que l'on isole et salifie si désiré, ou bien l'on soumet ledit produit de formule $I_C$ à l'action d'un agent d'halogénation pour obtenir un produit de formule $I_F$

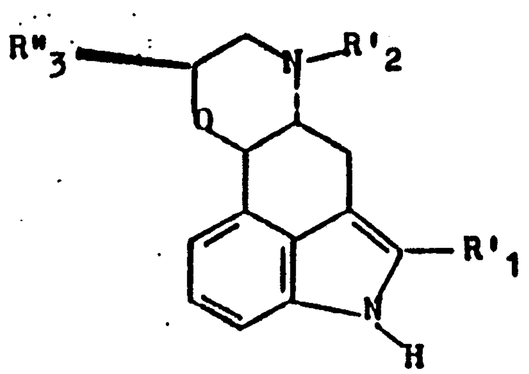

($I_F$)

dans laquelle R'$_1$, R''$_3$ et R'$_2$ ont la signification déjà indiquée, que l'on isole et si désiré, salifie.

6. Procédé selon la revendication 5, caractérisé en ce que le produit de formule II est préparé par réaction d'un produit de formule VII :

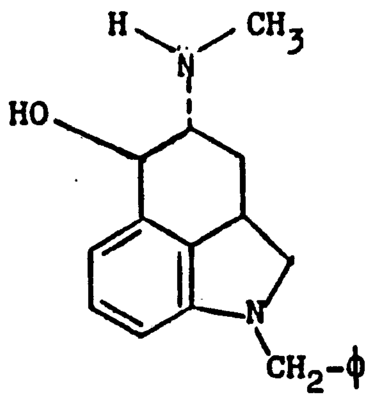

(VII)

avec un glycidate d'alcoyle de formule VIII

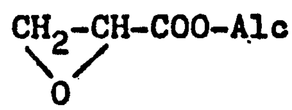

(VIII)

dans laquelle Alc représente un radical alcoyle renfermant de 1 à 4 atomes de carbone, pour obtenir un produit de formule IX

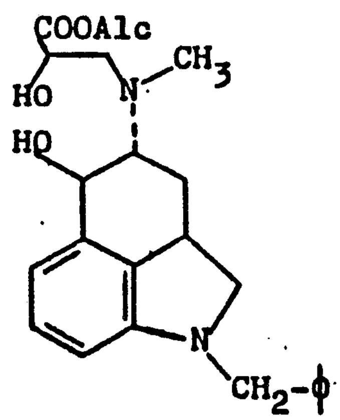

(IX)

dans laquelle Alc a la signification déjà indiquée, que l'on cyclise pour obtenir un produit de formule X

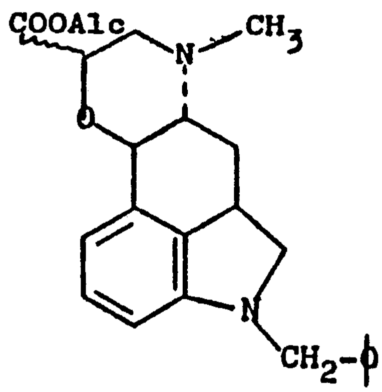

(X)

dans laquelle Alc a la signification déjà indiquée, et le trait ondulé signifie que le substituant est en position 9α ou 9β, dont on épimérise l'isomère 9α, puis estérifie l'acide ainsi obtenu, dans lequel le substituant en 9 est en position 9β pour obtenir ledit produit de formule II.

7. Médicaments, caractérisés en ce qu'ils sont constitués par les nouveaux dérivés de l'acide 9-oxalysergique, tels que définis par la formule I de la revendication 1, ainsi que par leurs sels d'addition avec les acides pharmaceutiquement acceptables.

8. Médicaments, caractérisés en ce qu'ils sont constitués par les nouveaux dérivés de l'acide 9-oxalysergique, tels que définis dans l'une des revendications 2 ou 3, ainsi que par leurs sels d'addition avec les acides pharmaceutiquement acceptables.

9. Médicaments, caractérisés en ce qu'ils sont constitués par le dérivé de l'acide 9-oxalysergique, tel que défini à la revendication 4, ainsi que par ses sels d'addition avec les acides pharmaceutiquement acceptables.

10. Compositions pharmaceutiques, caractérisées en ce qu'elles renferment, à titre de principe actif l'un au moins des médicaments, tels que définis à l'une des revendications 7, 8 ou 9.

**Revendications** pour l'Etat contractant AT

1.- Procédé de préparation des nouveaux dérivés de l'acide 9-oxalysergique, ainsi que de leurs sels, répondant à la formule générale (I):

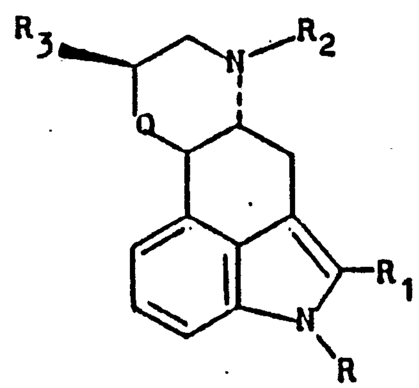

(I)

dans laquelle R représente un atome d'hydrogène ou un radical alcoyle renfermant de 1 à 4 atomes de carbone, $R_1$ représente un atome d'hydrogène de chlore ou de brome, $R_2$ représente un atome d'hydrogène, un radical alcoyle renfermant de 1 à 4 atomes de carbone, benzyle ou phénéthyle éventuellement substitués par un ou plusieurs substituants choisis dans la groupe des atomes d'halogène, des radicaux méthyl, étlyl, méthoxy, hydroxy ou trifluorométhyl ou cycloalcoylalcoyle renfermant de 4 à 7 atomes de carbone et $R_3$ représente un radical alcoyl-thiométhyle, dont l'alcoyle renferme de 1 à 3 atomes de carbone et est linéaire, caractérisé en ce que l'on débenzyle le produit de formule (II):

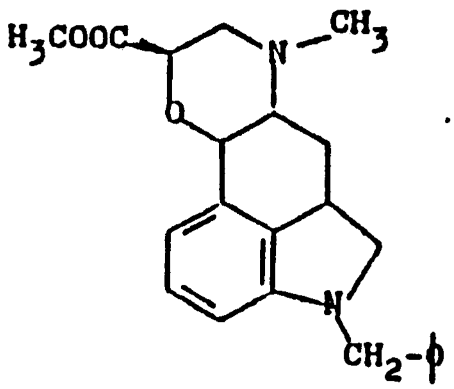

(II)

pour obtenir le produit de formule III

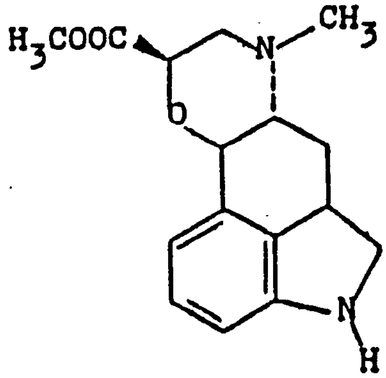

(III)

que l'on soumet à une déshydrogénation pour obtenir le produit de formule ($I_A$):

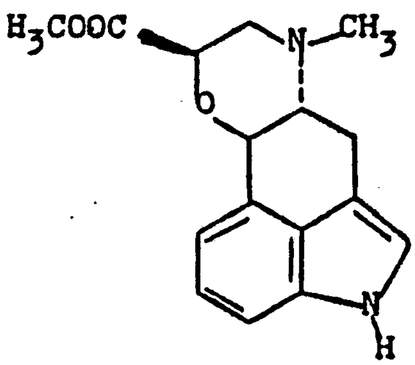

($I_A$)

que l'on réduit pour obtenir un produit de formule ($I_J$) :

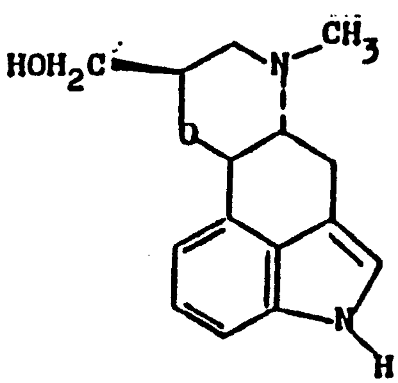

($I_J$)

que l'on fait réagir avec le chlorure de méthane sulfonyle ou le chlorure de paratoluène sulfonyle pour obtenir un produit de formule (VI):

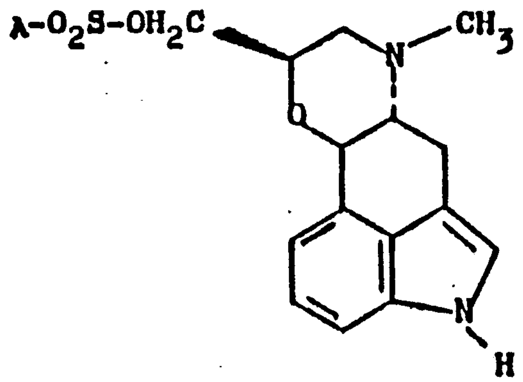

(VI)

dans laqualle A représente un radical méthyle ou para-tolyle, que l'on fait réagir avec un alcoylmercaptan, pour obtenir un produit de formule ($I_K$):

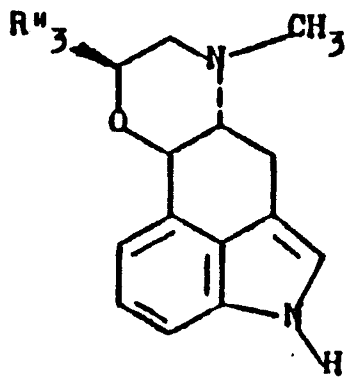

($I_K$)

dans laquelle R''$_3$ représente un radical alcoylthiométhyle que soit l'on isole et si désiré, salifie que soit l'on déméthyle pour obtenir le produit de formule $I_B$

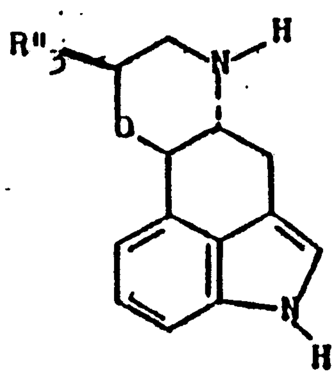

($I_B$)

dans laquelle R''$_3$ a la signification déjà indiquée, que soit l'on isole et si désiré salifie, soit l'on fait réagir avec un agent d'alcylation pour obenir un produit de formule $I_C$

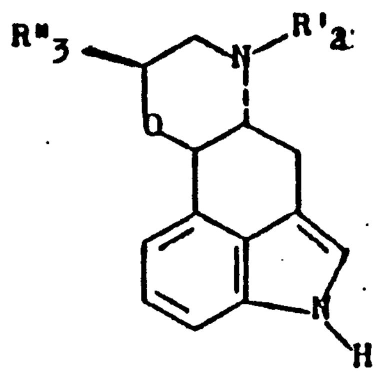

($I_C$)

dans laquelle R''$_3$ a la signification déjà indiquée et

R'$_2$ a la signification de $R_2$ déjà indiquée à l'exception de l'hydrogène, que ou bien l'on isole, et, si désiré, salifie, ou bien l'on fait réagir avec un amidure alcalin, puis avec un halogénure d'alcoyle de formule IV

Hal-R' (IV)

dans laquelle Hal représente un atome de chlore, de brome, ou d'iode, et R' a la signification de R déjà indiquée, à l'exception de l'hydrogène, pour obtenir un produit de formule $I_D$

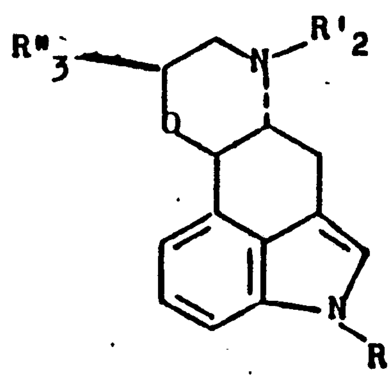

($I_D$)

dans laquelle R', R''$_3$ et R'$_2$ ont la signification déjà indiquée que soit l'on isole et si désiré, salifie, soit l'on soumet à l'action d'un agent d'halogénation pour obtenir un produit de formule $I_E$

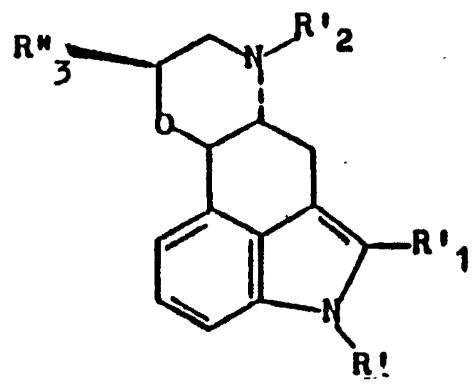

($I_E$)

dans laquelle R', R''$_3$ et R'$_2$ ont la signification déjà indiquée et R'$_1$ représente un atome de chlore ou de brome que l'on isole et salifie si désiré, ou bien l'on soumet ledit produit de formule $I_C$ à l'action d'un agent d'halogénation pour obtenir un produit de formule $I_F$

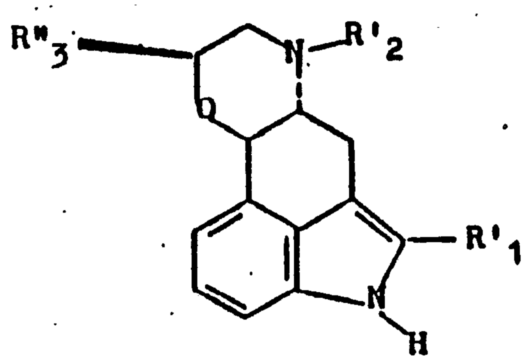

$(I_F)$

dans laquelle $R'_1$,$R''_3$ et $R'_2$ ont la signification déjà indiquée, que l'on isole et si désiré, salifie.

2. Procédé selon la revendication 1, caractérisé en ce que le produit de formule II est preparé par réaction d'un produit de formule VII:

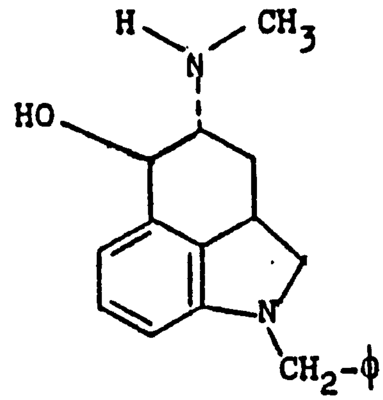

(VII)

avec un glycidate d'alcoyle de formule VIII

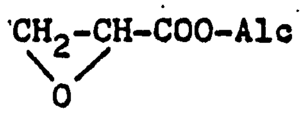

(VIII)

dans laquelle Alc représente un radical alcoyle renfermant de 1 à 4 atomes de carbone pour obtenir un produit de formule IX

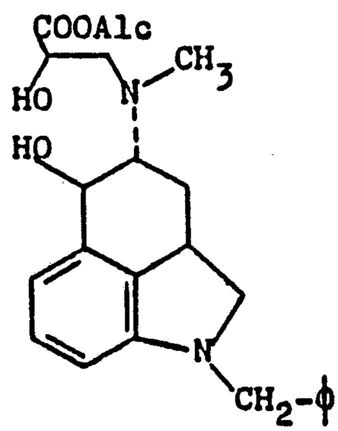

(IX)

dans laquelle Alc a la signification déjà indiquée, que l'on cyclise pour obtenir un produit de formule X

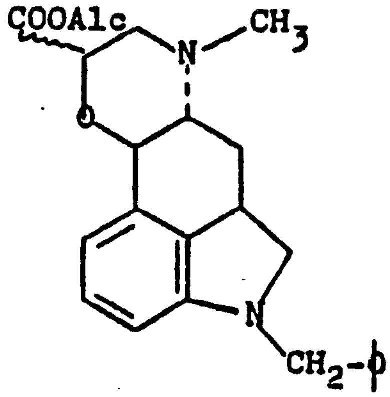

(X)

dans laquelle Alc a la signification déjà indiquée, et le trait ondulé signifie que le substituant est en position 9α ou 9β, dont on épimérise l'isomère 9α, puis estérifie l'acide ainsi obtenu, dans lequel le substituant en 9 est en position 9β pour obtenir ledit produit de formule II.

3. Procédé selon la revendication 1 ou 2, caractérisé en ce que l'on prépare des dérivés de l'acide 9-oxalysergique de formule I, ainsi que leurs sels, formule I dans laquelle R répresente un atome d'hydrogène.

4. Procédé selon la revendication 1 ou 2, caractérisé en ce que l'on prépare des dérivés de l'acide 9-oxalysergique de formule I, ainsi que leurs sels, formule I dans laquelle $R_1$ représente un atome d'hydrogène, de chlore ou de brome, $R_2$ représente un radical alcoyle renfermant de 1 à 4 atomes de carbone et $R_3$ représente un radical méthylthiométhyle.

5. Procédé selon la revendication 1 ou 2, caractérisé en ce que l'on prépare l'un qualconque des dérivés de formule I, telle que définie à la revendication 1, dont les noms suivent :

-1a /6a RS (6aα,9β, 10aβ)/4,6,6a,8,9 10a-hexahydro 7-méthyl 9-methylthiométhyl 7H-indolo (3,4-g,h) 1,4-benzoxazine, ainsi que ses sels d'addition avec les acides minéraux ou organiques.

**Patentansprüche**

für die Vertragsstaaten: BE, CH, DE, GB, IT, LI, LU, NL, SE

1. Neue Derivate der 9-Oxalysergsäure sowie deren Additionssalze mit Mineral- oder organischen Säuren, dadurch gekennzeichnet, daß sie der allgemeinen Formel I

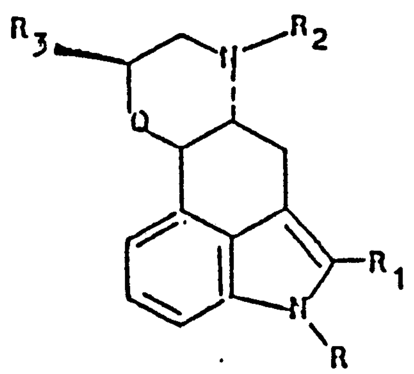

(I)

entsprechen, worin R ein Wasserstoffatom oder einen Alkylrest mit 1 bis 4 Kohlenstoffatomen bedeutet, $R_1$ für ein Wasserstoff-, Chlor- oder Bromatom steht, $R_2$ ein Wasserstoffatom, einen Alkylrest mit 1 bis 4 Kohlenstoffatomen, einen Benzyl- oder Phenethylrest, gegebenenfalls substituiert durch einen oder mehrere Substituenten, ausgewählt unter den Halogenatomen, den Methyl-, Ethyl-, Methoxy-, Hydroxy- oder Trifluormethylresten, oder einen Cycloalkalkylrest mit 4 bis 7 Kohlenstoffatomen bedeutet und $R_3$ für eine Alkylthiomethylgruppe steht, deren Alkylteil 1 bis 3 Kohlenstoffatome umfaßt und linear ist.

2. Neue Derivate der 9-Oxalysergsäure sowie deren Additionssalze mit Mineral- oder organischen Säuren, dadurch gekennzeichnet, daß sie der Formel I gemäß Anspruch 1 entsprechen, worin R ein Wasserstoffatom bedeutet.

3. Neue Derivate der 9-Oxalysergsäure gemäß Anspruch 2 sowie deren Additionssalze mit Mineral- oder organischen Säuren, dadurch gekennzeichnet, daß $R_1$ ein Wasserstoff-, Chlor- oder Bromatom bedeutet, $R_2$ für einen Alkylrest mit 1 bis 4 Kohlenstoffatomen steht und $R_3$ einen Methylthiomethylrest darstellt.

4. Produkt der Formel I gemäß Anspruch 1 mit der folgenden Bezeichnung
[6a-RS-(6aα,9β,10aβ)]-4,6,6a,8,9,10a-Hexa-hydro-7-methyl-9-methylthiomethyl-7H-indolo[3,4-g,h]-1,4-benzoxazin
sowie dessen Additionssalze mit Mineral- oder organischen Säuren.

5. Verfahren zur Herstellung der Derivate der 9-Oxalysergsäure der Formel I gemäß Anspruch 1 sowie von deren Salzen, dadurch gekennzeichnet, daß man das Produkt der Formel II

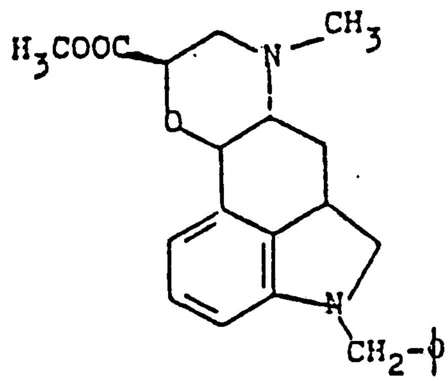

(II)

entbenzyliert, um das Produkt der Formel III

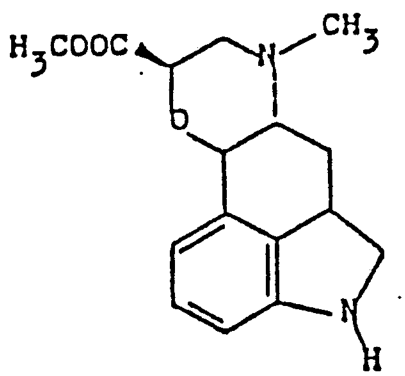

(III)

zu erhalten, das man einer Dehydrierung unterzieht, um das Produkt der Formel ($I_A$)

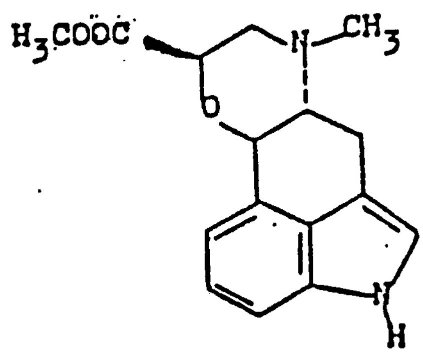

($I_A$)

zu erhalten, welches man reduziert, um ein Produkt der Formel ($I_J$)

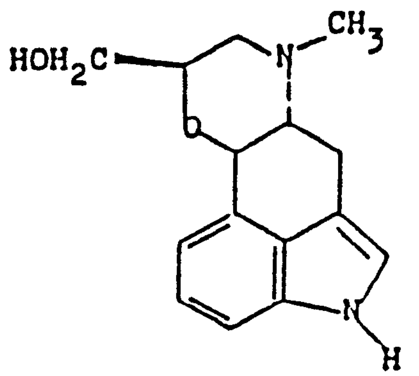

($I_J$)

zu erhalten, das man mit Methansulfonylchlorid oder p-Toluolsulfonylchlorid umsetzt, um ein Produkt der Formel (VI)

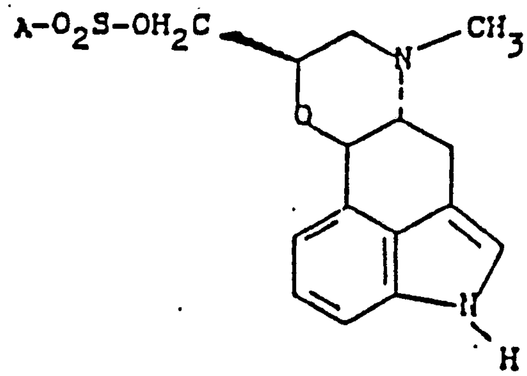

(VI)

zu erhalten, in der A einen Methyl- oder p-Tolylrest bedeutet, welches man mit einem Alkylmercaptan umsetzt, um ein Produkt der Formel ($I_K$)

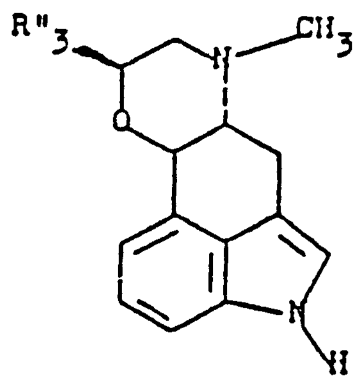

($I_K$)

zu erhalten, worin $R''_3$ einen Alkylthiomethylrest bedeutet, das man entweder isoliert und gewünschtenfalls in ein Salz überführt oder entmethyliert, um das Produkt der Formel $I_B$

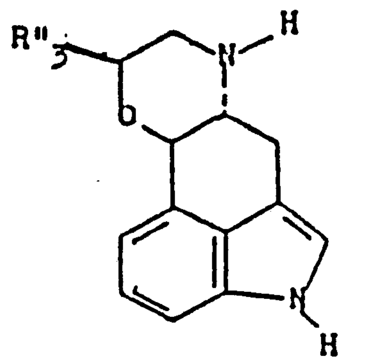

$(I_B)$

zu erhalten, worin $R''_3$ die angegebene Bedeutung besitzt, welches man entweder isoliert und gewünschtenfalls in ein Salz überführt oder mit einem Alkylierungsmittel umsetzt, um ein Produkt der Formel $I_C$

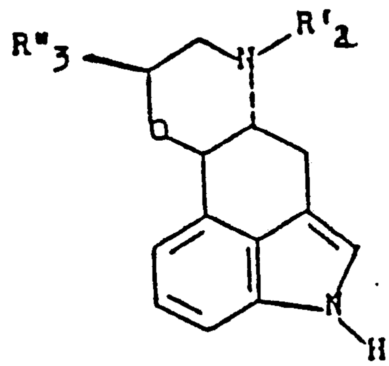

$(I_C)$

zu erhalten, worin $R''_3$ die angegebene Bedeutung besitzt und $R'_2$ die für $R_2$ angegebene Bedeutung mit Ausnahme von Wasserstoff besitzt, welches man entweder isoliert und gewünschtenfalls in ein Salz überführt oder mit einem Alkaliamid, danach mit einem Alkylhalogenid der Formel IV

Hal-R' (IV)

worin Hal ein Chlor-, Brom- oder Jodatom bedeutet und R' die für R angegebene Bedeutung mit Ausnahme von Wasserstoff besitzt, umsetzt, um ein Produkt der Formel $I_D$

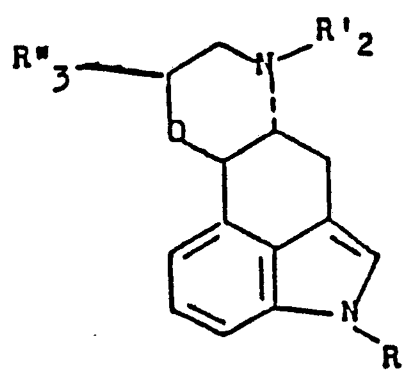

$(I_D)$

zu erhalten, worin R', $R''_3$ und $R'_2$ die angegebene Bedeutung besitzen, welches man entweder isoliert und gewünschtenfalls in ein Salz überführt oder der Einwirkung eines Halogenierungsmittels unterzieht, um ein Produkt der Formel $I_E$

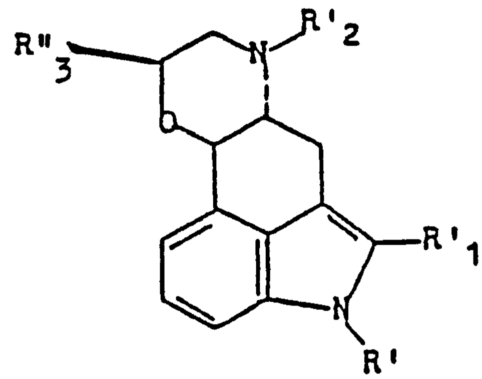

($I_E$)

zu erhalten, worin R', R''$_3$ und R'$_2$ die angegebene Bedeutung besitzen und R'$_1$ fur ein Chlor- oder Bromatom steht, welches man isoliert und gewünschtenfalls in ein Salz überführt, oder das Produkt der Formel $I_C$ der Einwirkung eines Halogenierungsmittels unterzieht, um ein Produkt der Formel $I_F$

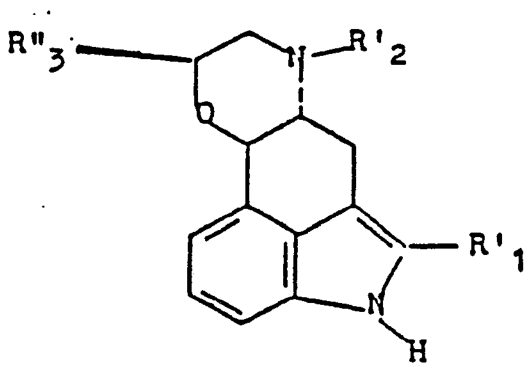

($I_F$)

zu erhalten, worin R'$_1$ ,R''$_3$ und R'$_2$ die angegebene Bedeutung besitzen, das man isoliert und gewünschtenfalls in ein Salz überführt.

6. Verfahren gemaß Anspruch 5, dadurch gekennzeichnet, daß die Herstellung des Produkts der Formel II durch Umsetzung eines Produkts der Formel VII

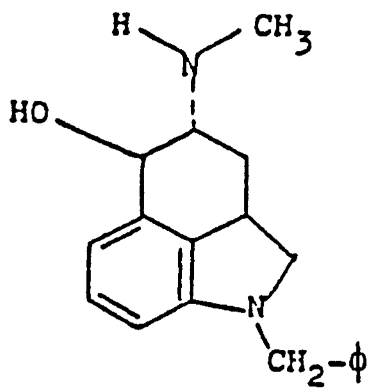

(VII)

mit einem Alkylglycidat der Formel VIII

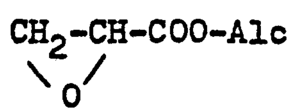

(VIII)

worin Alc für einen Alkylrest mit 1 bis 4 Kohlenstoffatomen steht, erfolgt, um ein Produkt der Formel IX

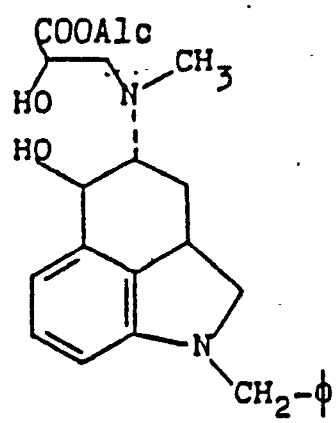

(IX)

zu erhalten, worin Alc die angegebene Bedeutung besitzt, welches man cyclisiert, um ein Produkt der Formel X

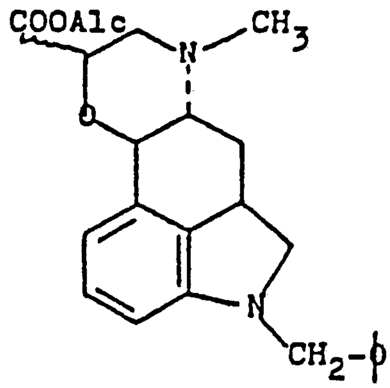

(X)

zu erhalten, worin Alc die angegebene Bedeutung besitzt und die gewellte Linie anzeigt, daß sich der Substituent in 9α- oder 9β-Stellung befindet, dessen 9α-Isomeres man epimerisiert und danach die so erhaltene Säure, in welcher der Substituent in 9-Stellung sich in 9β-Stellung befindet, verestert, um das Produkt der Formel II zu erhalten.

7. Arzneimittel, dadurch gekennzeichnet, daß sie aus den neuen Derivaten der 9-Oxalysergsäure der Formel I gemäß Anspruch 1 sowie aus deren Additionssalzen mit pharmazeutisch verträglichen Suren bestehen.

8. Arzneimittel, dadurch gekennzeichnet, daß sie aus den neuen Derivaten der 9-Oxalysergsäure gemäß einem der Ansprüche 2 oder 3 sowie aus deren Additionssalzen mit pharmazeutisch verträglichen Säuren bestehen.

9. Arzneimittel, dadurch gekennzeichnet, daß sie aus dem Derivat der 9-Oxalysergsäure gemäß Anspruch 4 sowie aus dessen Additionssalz mit pharmazeutisch vertraglichen Säuren bestehen.

10. Pharmazeutische Zusammensetzungen, dadurch gekennzeichnet, daß sie als Wirkstoff zumindest eines der Arzneimittel gemäß einem der Ansprüche 7, 8 oder 9 enthalten.

**Patentansprüche** für den Vertragsstaat: AT

1. Verfahren zur Herstellung der neuen Derivate der 9-Oxalysergsäure sowie von deren Salze der allgemeinen Formel (I)

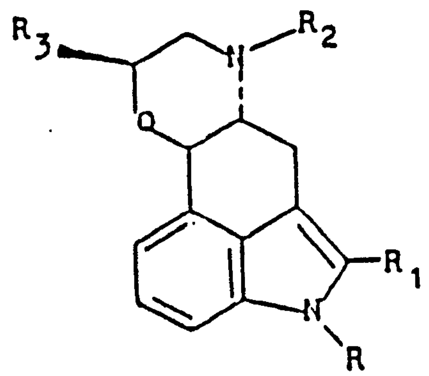

(I)

worin R ein Wasserstoffatom oder einen Alkylrest mit 1 bis 4 Kohlenstoffatomen bedeutet, $R_1$ fur ein Wasserstoff-, Chlor oder Bromatom steht, $R_2$ ein Wasserstoffatom, einen Alkylrest mit 1 bis 4 Kohlenstoffatomen, einen Benzyl- oder Phenethylrest, gegebenenfalls substituiert durch einen oder mehrere Substituenten, ausgewählt unter den Halogenatomen, den Methyl-, Ethyl-, Methoxy-, Hydroxy- oder Trifluormethylresten, oder einen Cycloalkalkylrest mit 4 bis 7 Kohlenstoffatomen darstellt und $R_3$ einen Alkylthiomethylrest, dessen Alkylteil 1 bis 3 Kohlenstoffatome umfaßt und linear ist, bedeutet,

dadurch gekennzeichnet, daß man das Produkt der Formel (II)

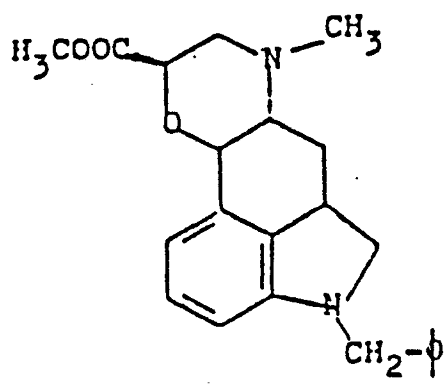

(II)

entbenzyliert, um das Produkt der Formel III

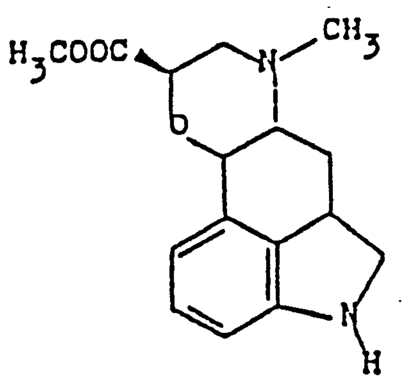

(III)

zu erhalten, das man einer Dehydrierung unterzieht, um das Produkt der Formel ($I_A$)

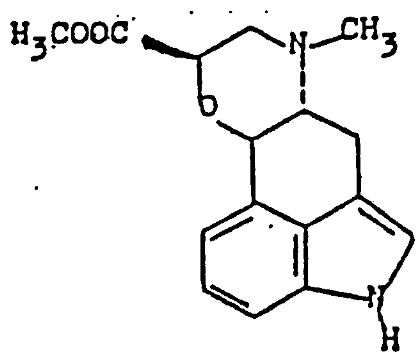

($I_A$)

zu erhalten, welches man reduziert, um ein Produkt der Formel ($I_J$)

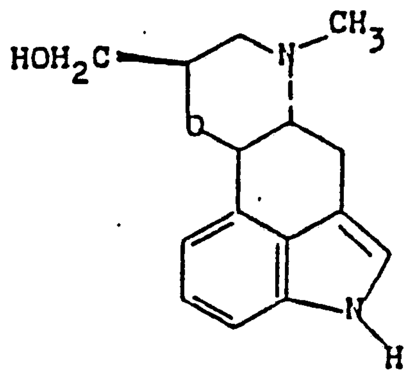

($I_J$)

zu erhalten, das man mit Methansulfonylchlorid oder p-Toluolsulfonylchlorid umsetzt, um ein Produkt der Formel (VI)

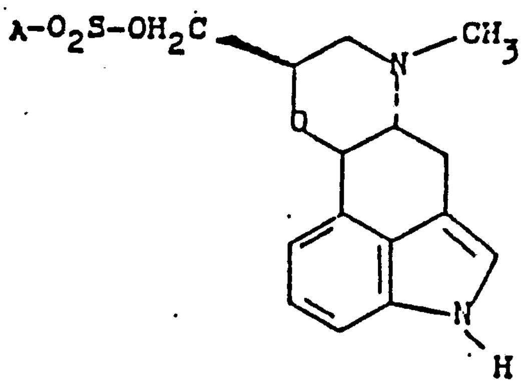

(VI)

zu erhalten, in der A einen Methyl- oder p-Tolylrest bedeutet, welches man mit einem Alkylmercaptan umsetzt, um ein Produkt der Formel ($I_K$)

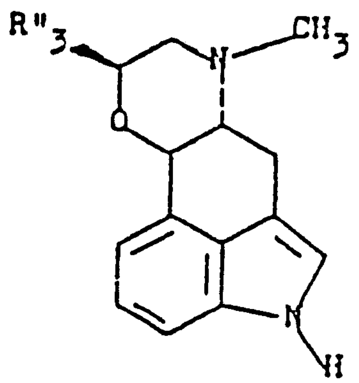

(I$_K$)

zu erhalten, worin $R''_3$ einen Alkylthiomethylrest bedeutet, das man entweder isoliert und gewünschtenfalls in ein Salz überführt oder entmethyliert, um das Produkt der Formel $I_B$

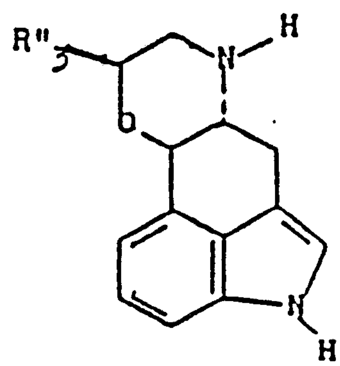

(I$_B$)

zu erhalten, worin $R''_3$ die angegebene Bedeutung besitzt, welches man entweder isoliert und gewünschtenfalls in ein Salz überführt oder mit einem Alkylierungsmittel umsetzt, um ein Produkt der Formel $I_C$

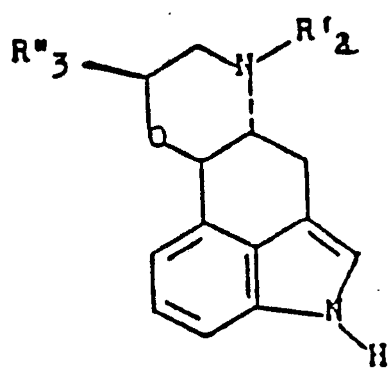

(I$_C$)

zu erhalten, worin $R''_3$ die angegebene Bedeutung besitzt und $R'_2$ die für $R_2$ angegebene Bedeutung mit Ausnahme von Wasserstoff besitzt, welches man entweder isoliert und gewünschtenfalls in ein Salz überführt oder mit einem Alkaliamid, danach mit einem Alkylhalogenid der Formel IV

Hal-R' (IV)

worin Hal ein Chlor-, Brom- oder Jodatom bedeutet und R' die für R angegebene Bedeutung mit Ausnahme von Wasserstoff besitzt, umsetzt, um ein Produkt der Formel $I_D$

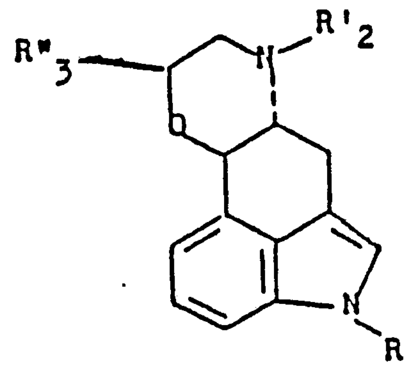

$(I_D)$

zu erhalten, worin R' , R''$_3$ und R'$_2$ die angegebene Bedeutung besitzen, welches man entweder isoliert und gewünschtenfalls in ein Salz überführt oder der Einwirkung eines Halogenierungsmittels unterzieht, um ein Produkt der Formel $I_E$

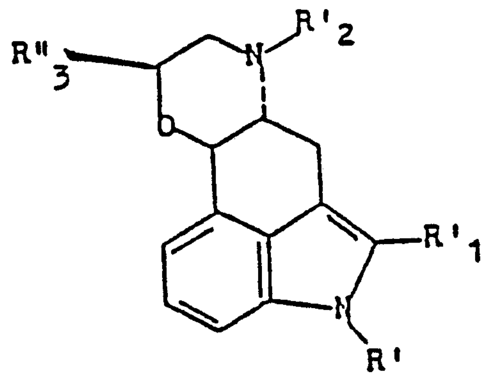

$(I_E)$

zu erhalten, worin R' , R''$_3$ und R'$_2$ die angegebene Bedeutung besitzen und R'$_1$ für ein Chlor- oder Bromatom steht, welches man isoliert und gewünschtenfalls in ein Salz überführt, oder das Produkt der Formel $I_C$ der Einwirkung eines Halogenierungsmittels unterzieht, um ein Produkt der Formel $I_F$

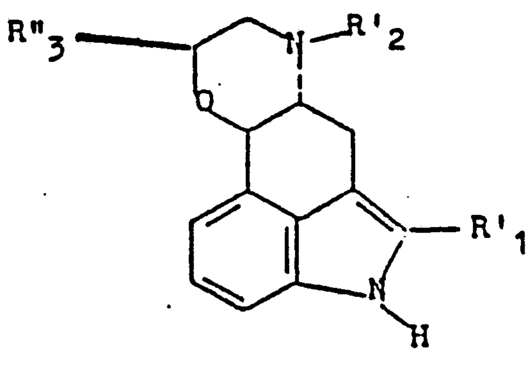

$(I_F)$

zu erhalten, worin R'$_1$ , R''$_3$ und R'$_2$ die angegebene Bedeutung haben, das man isoliert und gewünschtenfalls in ein Salz überführt.

2. Verfahren gemäß Anspruch 1, dadurch gekennzeichnet, daß die Herstellung des Produkts der Formel II durch Umsetzung eines Produkts der Formel VII

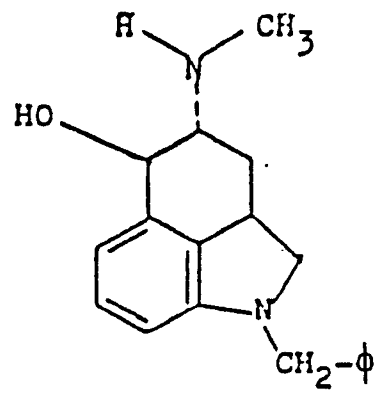

(VII)

mit einem Alkylglycidat der Formel VIII

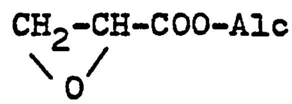

(VIII)

worin Alc für einen Alkylrest mit 1 bis 4 Kohlenstoffatomen steht, erfolgt, um ein Produkt der Formel IX

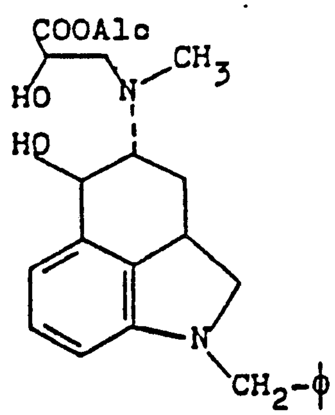

(IX)

zu erhalten, worin Alc die angegebene Bedeutung besitzt, welches man cyclisiert, um ein Produkt der Formel X

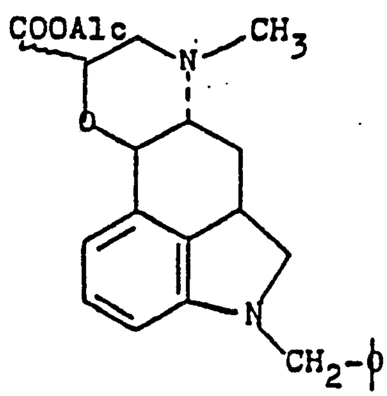

(X)

zu erhalten, worin Alc die angegebene Bedeutung besitzt und die gewellte Linie anzeigt, daß sich der Substituent in 9α- oder 9β-Stellung befindet, dessen 9α-Isomeres man epimerisiert, danach die so erhaltene Säure, in der der Substituent in 9-Stellung sich in 9β-Stellung befindet, verestert, um das Produkt der Formel II zu erhalten.

3. Verfahren gemäß Anspruch 1 oder 2, dadurch gekennzeichnet, daß man die Derivate der 9-Oxalysergsäure der Formel I sowie deren Salze herstellt, worin R ein Wasserstoffatom bedeutet.

4. Verfahren gemäß Anspruch 1 oder 2, dadurch gekennzeichnet, daß man die Derivate der 9-Oxalysergsäure der Formel I sowie deren Salze herstellt, worin $R_1$ ein Wasserstoff-, Chlor- oder Bromatom bedeutet, $R_2$

für einen Alkylrest mit 1 bis 4 Kohlenstoffatomen steht und $R_3$ einen Methylthiomethylrest darstellt.

5. Verfahren gemäß Anspruch 1 oder 2, dadurch gekennzeichnet, daß man das Derivat der Formel I gemäß Anspruch 1 mit der folgenden Bezeichnung herstellt

[6a-RS-(6aα,9β,10aβ)]-4,6,6a,8,9,10a-Hexahydro-7-methyl-9-methylthiomethyl-7H-indolo[3,4-g,h]-1,4-benzoxazin

sowie dessen Additionssalz mit Mineral- oder organischen Säuren.

**Claims** for the contracting states: BE CH DE GB IT LI LU NL SE

1) New derivatives of 9-oxalysergic acid, as well as their addition salts with mineral or organic acids, characterized in that they answer to the general formula (I):

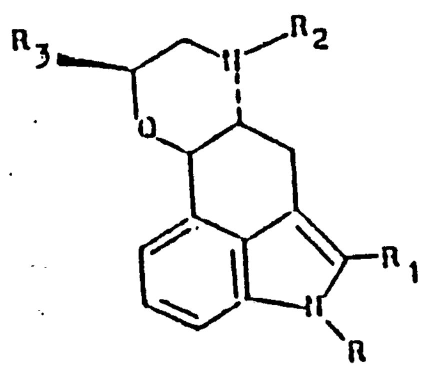

(I)

in which R represents a hydrogen atom or an alkyl radical containing from 1 to 4 carbon atoms, $R_1$ represents a hydrogen, chlorine or bromine atom, $R_2$ represents a hydrogen atom, an alkyl radical containing from 1 to 4 carbon atoms, benzyl or phenethyl possibly substituted by one or more substituents chosen from the group of halogen atoms, and the following radicals:- methyl, ethyl, methoxy, hydroxy or trifluoromethyl or cycloalkylalkyl containing from 4 to 7 carbon atoms and $R_3$ represents an alkyl-thiomethyl radical, of which the alkyl contains from to 3 carbon atoms and is linear.

2) New derivatives of 9-oxalysergic acid, as well as their addition salts with mineral or organic acids, characterized in that they answer to the formula (I) of claim 1, In which R represents a hydrogen atom.

3) New derivatives of 9-oxalysergic acid according to claim 2, as well as their addition salts with mineral or organic acids, characterized in that $R_1$ represents a hydrogen, chlorine or bromine atom, $R_2$ represents an alkyl radical containing from 1 to 4 carbon atoms and $R_3$ represents a methylthiomethyl radical.

4) The product with the formula (I) according to claim 1 of which the name follows:

[6a RS (6a-alpha,9-beta,10a-beta)] 4,6,6a,8,g,10a-hexahydro-7-methyl-9-methylthiomethyl-7H-indolo[3,4-g,h]-1,4-benzoxazine, as well as its addition salts with mineral or organic acids.

5) Preparation process for derivatives of 9-oxalysergic acid as defined by the formula (I) of claim 1, as well as their salts, characterized in that the product with the formula (II):

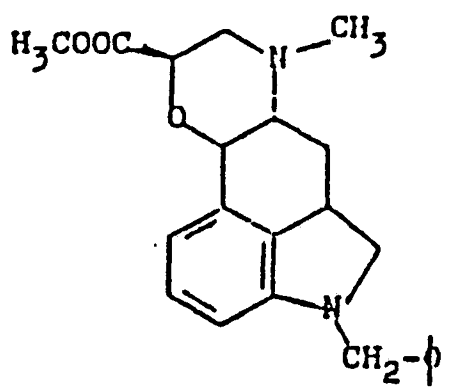

(II)

is debenzylated in order to obtain the product with the formula (III):

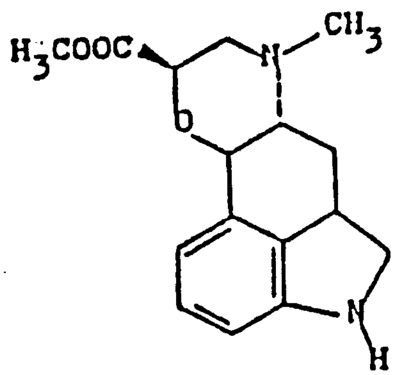

(III)

which is submitted to a dehydrogenation in order to obtain the product with the formula ($I_A$):

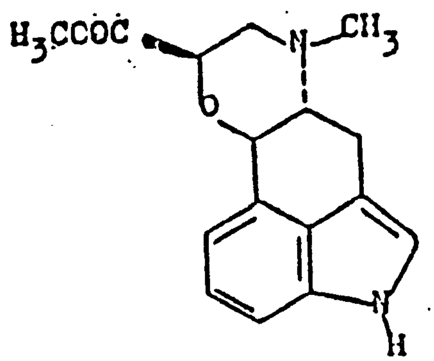

($I_A$)

which is reduced in order to obtain a product with the formula ($I_J$):

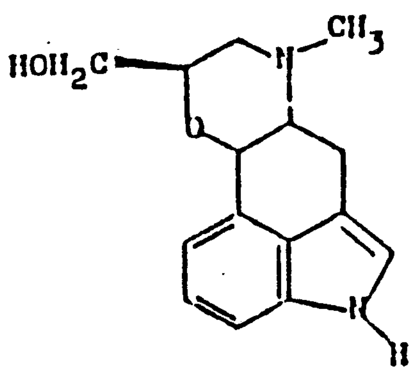

($I_J$)

which is made to react with methane sulphonyl chloride or paratoluene sulphonyl chloride in order to obtain a product with the formula (VI):

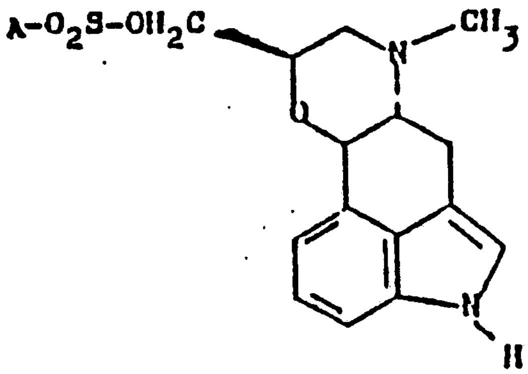

(VI)

in which A represents a methyl or paratolyl radical, which is made to react with an alkylmercaptan, in order to obtain a product with the formula ($I_K$):

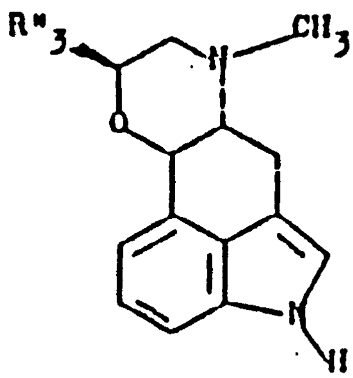

($I_K$)

in which R"$_3$ represents an alkylthiomethyl radical which either is isolated and, if desired, salified, or is demethylated in order to obtain the product with the formula ($I_B$):

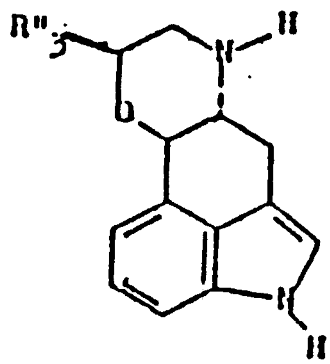

($I_B$)

in which R"$_3$ has the significance already indicated, which either is isolated and, if desired, salified, or is made to react with an alkylation agent in order to obtain a product with the formula ($I_C$):

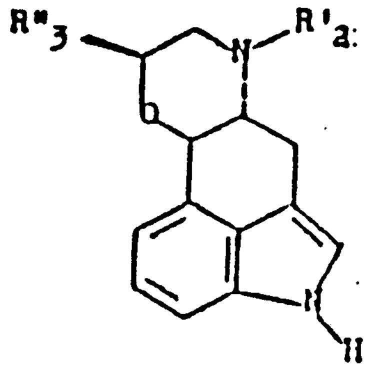

$(I_C)$

in which $R''_3$ has the significance already indicated and $R'_2$ has the significance of $R_2$ already indicated with the exception of hydrogen, which either is isolated, and, if desired, salified, or is made to react with an alkaline amide, then with an alkyl halogenide with the formula (IV):

Hal-R' (IV)

in which Hal represents a chlorine, bromine or iodine atom, and R' has the significance of R already indicated, with the exception of hydrogen, in order to obtain a product with the formula $(I_D)$:

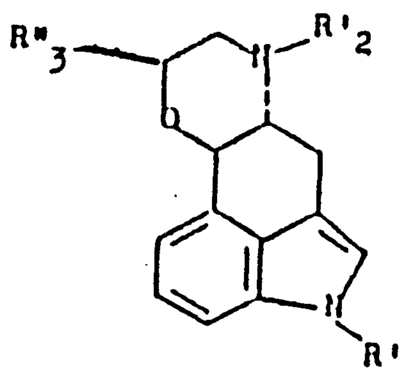

$(I_D)$

in which R', $R''_3$ and $R'_2$ have the significance already indicated, which either is isolated and, if desired, salified, or is submitted to the action of a halogenation agent in order to obtain a product with the formula $(I_E)$:

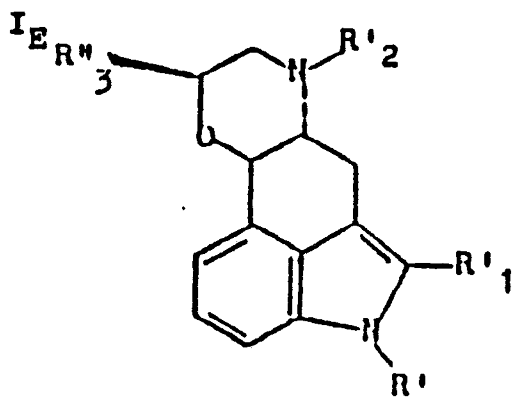

$(I_E)$

in which R', $R''_3$ and $R'_2$ have the significance already indicated and $R'_1$ represents a chlorine or bromine atom, which is isolated and salified if desired, or the said product with the formula $(I_C)$ is submitted to the action of a halogenation agent in order to obtain a product with the formula $(I_F)$:

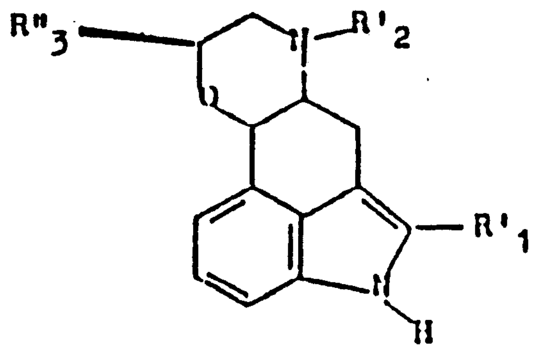

$(I_F)$

in which $R'_1$, $R''_3$ and $R'_2$ have the significance already indicated, which is isolated and, if desired, salified.

6) Process according to claim 5, characterized in that the product with the formula (II) is prepared by reaction of a product with the formula (VII):

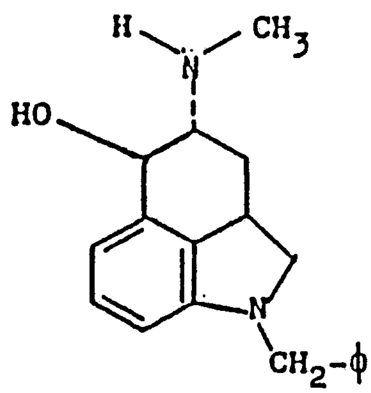

(VII)

with an alkyl glycidate with the formula (VIII):

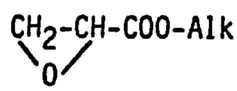

(VIII)

in which Alk represents an alkyl radical containing from 1 to 4 carbon atoms, in order to obtain a product with the formula (IX):

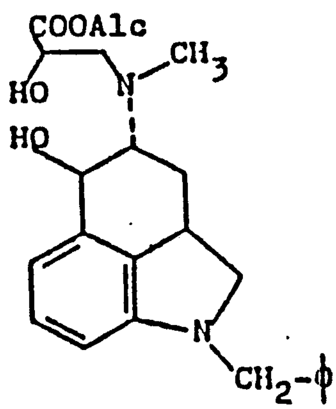

(IX)

in which Alk has the significance already indicated, which is cyclized in order to obtain a product with the formula (X):

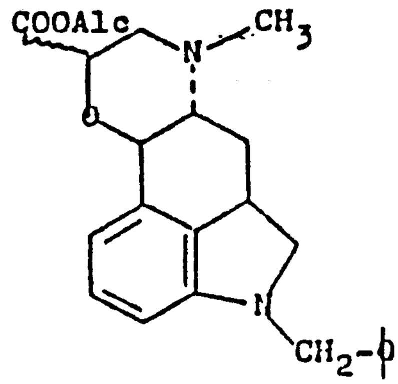

(X)

in which Alk has the significance already indicated, and the wavy line signifies that the substituent is in the 9-alpha or 9-beta position, the 9-alpha isomer of which is epimerized, then the acid thus obtained in which the substituent in the 9 position is in the 9-beta position, is esterified in order to obtain the said product with the formula (II).

7) Medicaments, characterized in that they are constituted by the new derivatives of 9-oxalysergic acid, as defined by the formula (I) of claim 1, as well as by their addition salts with pharmaceutically acceptable acids.

8) Medicaments, characterized in that they are constituted by the new derivatives of 9-oxalysergic acid, as defined in one of the claims 2 or 3, as well as by their addition salts with pharmaceutically acceptable acids.

9) Medicaments, characterized in that they are constituted by the derivative of 9-oxalysergic acid, as defined in claim 4, as well as by its addition salts with pharmaceutically acceptable acids.

10) Pharmaceutical compositions, characterized in that they contain, as active principle, at least one of the medicaments as defined in one of the claims 7, 8 or 9.

**Claims** for the contracting state: AT

1) Preparation process for the new derivatives of 9-oxalysergic acid, as well as their salts, answering to the general formula (I).:

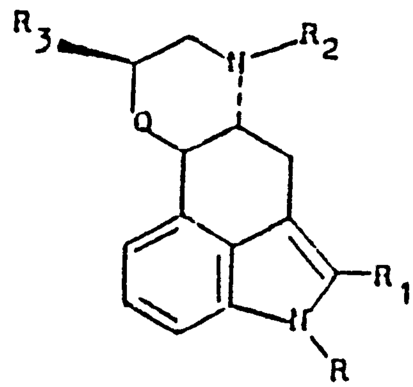

(I)

in which R represents a hydrogen atom or an alkyl radical containing from 1 to 4 carbon atoms, $R_1$ represents a hydrogen, chlorine or bromine atom, $R_2$ represents a hydrogen atom, an alkyl radical containing from 1 to 4 carbon atoms, benzyl or phenethyl possibly substituted by one or more substituents chosen from the group of halogen atoms, and the following radicals:- methyl, ethyl, methoxy, hydroxy or trifluoromethyl or cycloalkylalkyl containing from 4 to 7 carbon atoms and $R_3$ represents an alkyl-thiomethyl radical, of which the alkyl contains from to 3 carbon atoms and is linear, characterized in that the product with the formula (II):

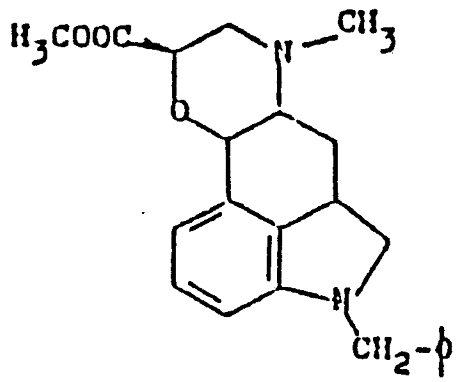

(II)

is debenzylated in order to obtain the product with the formula (III):

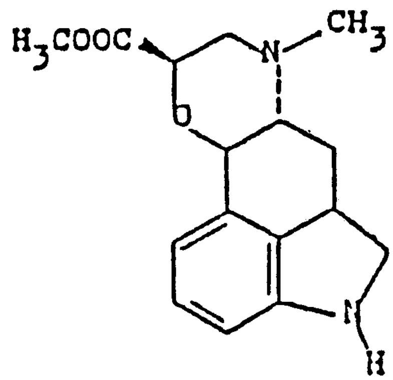

(III)

which is submitted to a dehydrogenation in order to obtain the product with the formula ($I_A$):

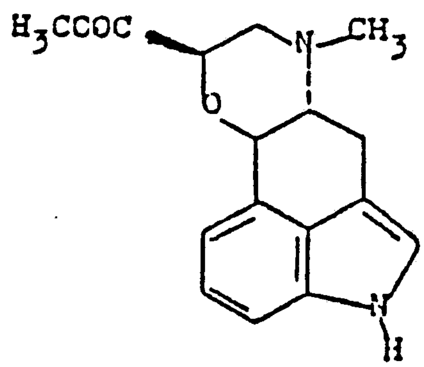

($I_A$)

which is reduced in order to obtain a product with the formula ($I_J$):

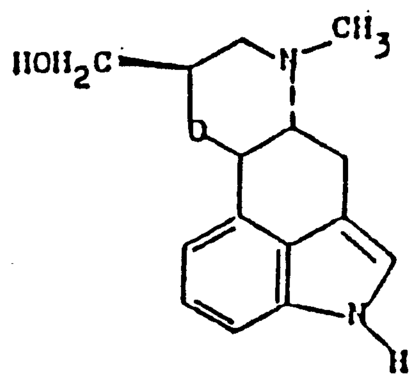

($I_J$)

which is made to react with methane sulphonyl chloride or paratoluene sulphonyl chloride in order to obtain a product with the formula (VI):

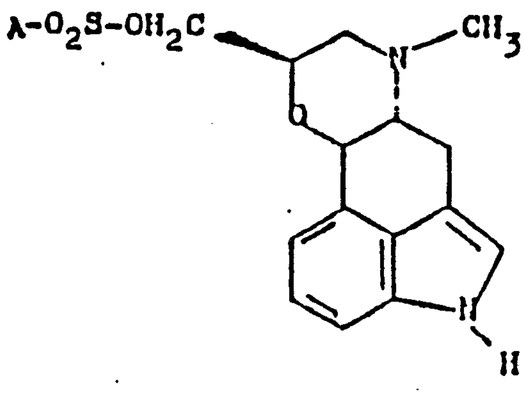

(VI)

in which A represents a methyl or paratolyl radical, which is made to react with an alkylmercaptan, in order to obtain a product with the formula ($I_K$):

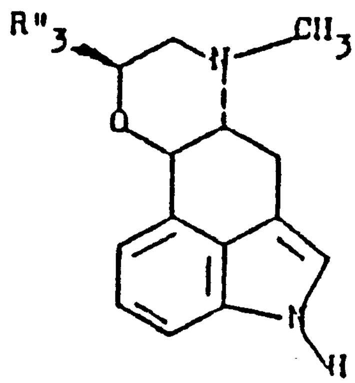

($I_K$)

in which $R''_3$ represents an alkylthiomethyl radical which either is isolated and, if desired, salified, or is demethylated in order to obtain the product with the formula ($I_B$):

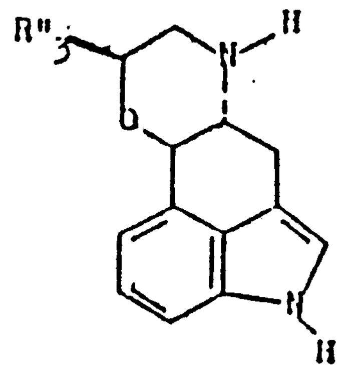

$(I_B)$

in which $R''_3$ has the significance already indicated, which either is isolated and, if desired, salified, or is made to react with an alkylation agent in order to obtain a product with the formula $(I_C)$:

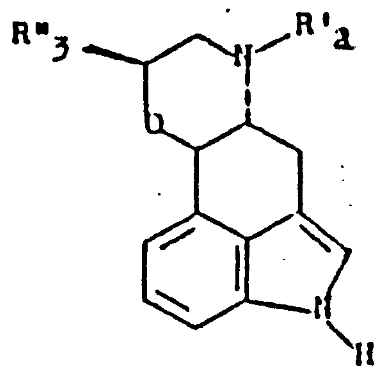

$(I_C)$

in which $R''_3$ has the significance already indicated and $R'_2$ has the significance of $R_2$ already indicated with the exception of hydrogen, which either is isolated and, if desired, salified, or is made to react with an alkaline amide, then with an alkyl halogenide with the formula (IV):

Hal-R' (IV)

in which Hal represents a chlorine, bromine or iodine atom, and R' has the significance of R already indicated, with the exception of hydrogen, in order to obtain a product with the formula $(I_D)$:

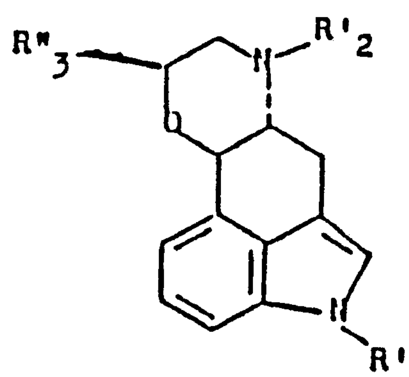

$(I_D)$

in which R', $R''_3$ and $R'_2$ have the significance already indicated, which either is isolated and, if desired, salified, or is submitted to the action of a halogenation agent in order to obtain a product with the formula $(I_E)$:

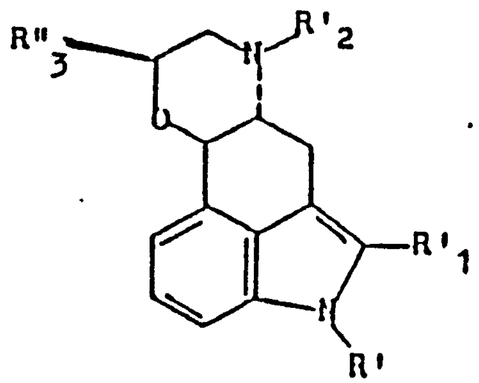

(IE)

in which R', R"$_3$ and R'$_2$ have the significance already indicated and R'$_1$ represents a chlorine or bromine atom, which is isolated and salified, if desired, <u>or</u> the said product with the formula ($I_C$) is submitted to the action of a halogenation agent in order to obtain a product with the formula ($I_F$):

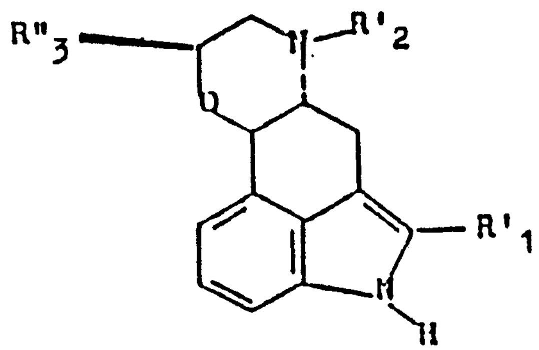

(IF)

in which R'$_1$, R"$_3$ and R'$_2$ have the significance already indicated, which is isolated and, if desired, salified.

2) Process according to claim 1, characterized in that the product with the formula (II) is prepared by reaction of a product with the formula (VII):

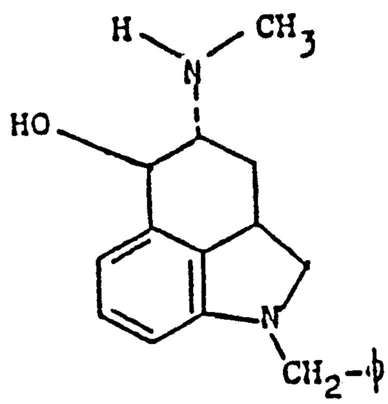

(VII)

with an alkyl glycidate with the formula (VIII):

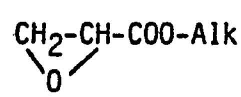

(VIII)

in which Alk represents an alkyl radical containing from 1 to 4 carbon atoms, in order to obtain a product with the formula (IX):

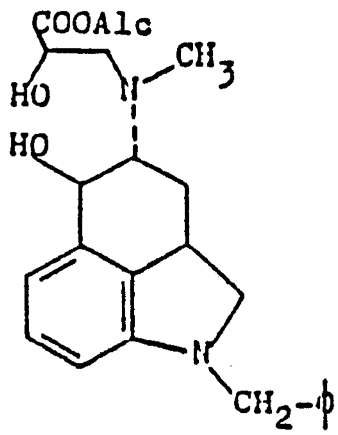

(IX)

in which Alk has the significance already indicated, which is cyclized in order to obtain a product with the formula (X):

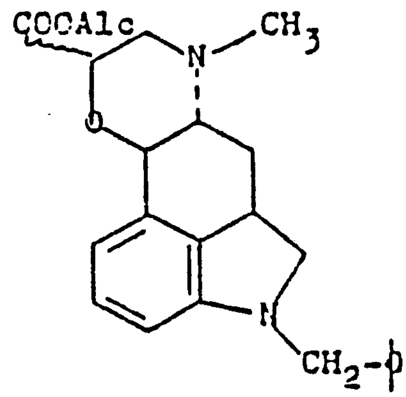

(X)

in which Alk has the significance already indicated, and the wavy line signifies that the substituent is in the 9-alpha or 9-beta position, the 9-alpha isomer of which is epimerized, then the acid thus obtained in which the substituent in the 9 position is in the 9-beta position, is esterified in order to obtain the said product with the formula (II).

3) Process according to claim 1 or 2, characterized in that the derivatives of 9-oxalysergic acid with the formula (I), as well as their salts, are prepared, in which formula (I) R represents a hydrogen atom.

4) Process according to claim 1 or 2, characterized in that the derivatives of 9-oxalysergic acid with the formula (I), as well as their salts, are prepared, in which formula (1) $R_1$ represents a hydrogen, chlorine or bromine atom, $R_2$ represents an alkyl radical containing from 1 to 4 carbon atoms and $R_3$ represents a methylthiomethyl radical.

5) Process according to claim 1 or 2, characterized in that any one of the derivatives with the formula (I) is prepared, as defined in claim 1, of which the names follow.:

[6a RS (6a-alpha,9-beta,10a-beta)] 4,6,6a,8,9,10a-hexahydro-7-methyl-9-methylthiomethy-7H-indolo[3,4-g,h]-1,4-benzoxazine, as well as its addition salts with mineral or organic acids.